# EUROPEAN PATENT SPECIFICATION

(11) **EP 3 016 643 B1**
(45) Date of publication and mention of the grant of the patent: **07.03.2018**
(21) Application number: 14731289.6
(22) Date of filing: 19.06.2014
(51) Int. Cl.: A61K 31/01, A61K 31/366, A61K 31/593, A61K 36/06, A61P 9/00

(54) **COMPOSITION HAVING A BENEFICIAL EFFECT ON THE CARDIOVASCULAR SYSTEM, COMPRISING MONACOLIN K, LYCOPENE AND VITAMIN D3**
ZUSAMMENSETZUNG ZUR ORALEN VERABREICHUNG MIT GÜNSTIGER WIRKUNG AUF DAS KARDIOVASKULARE SYSTEM, UMFASSEND EINE MISCHUNG VON MONACOLIN K, LYCOPEN UND VITAMIN D3.
COMPOSITION AYANT UN EFFET AVANTAGEUX SUR LE SYSTÈME CARDIOVASCULAIRE, COMPRENANT UN MÉLANGE DE MONACOLINE K, DE LYCOPÈNE ET DE VITAMINE D3.

(30) Priority: 21.06.2013 EP 13173297
(43) Date of publication of application: 11.05.2016
(73) Proprietor: Essential IP BV, 1404 AK Bussum (NL)
(72) Inventor: EIJGELAAR, Wouter-Jan, NL-3824 VP Amersfoort (NL); SCHOEVERS, Peter Alexander, NL-1404 AK Bussum (NL); VIËTOR, Hendrik Engelbertus, NL-1404 AK Bussum (NL)
(74) Representative: Life Science Patents B.V.
(86) International application number: PCT/EP2014/062964
(87) International publication number: WO 2014/202734

(56) References cited:
- US-A1- 2002 172 721
- US-A1- 2012 177 631

## Description

### Field of the invention

The present invention is in the field of medical treatment and relates to a formulation for oral administration, such as for instance in the form of tablets or of a powder, which is able to exert a beneficial effect on the cardiovascular system.

### Background of the invention

In the industrialized countries, cardiovascular diseases are among the main causes of death for men and women [Schwartz LM, Woloshin S: Changing disease definitions: implications for disease prevalence, Analysis of the Third National Health and Nutrition Examination Survey, 1988-1994. Eff Clin Pract 1999; 2:76-85, Lowe LP, Greenland P, Ruth KJ et al.: Impact of major cardiovascular disease risk factors, particularly in combination, on 22-year mortality in women and men. Arch Intern Med 1998; 158:2007-2014, Sempos CT, Ceeman JI, Carroll MD et al.: Prevalence of high blood cholesterol among US adults. An update based on guidelines from the second report of the National Cholesterol Education Program Adult Treatment Panel. JAMA 1993; 269:3009-3014].

Many cardiovascular diseases are of an atherosclerotic nature, i.e. are due to narrowing of the lumen of the arteries owing to the deposition of cholesterol, calcium and fibrin in the vessel wall, or to occlusion of the arteries caused by a thrombus that formed from an atheromatous plaque. As was documented in the Framingham epidemiological study [Kannel WB, Castelli WP, Gordon T et al. Lipoprotein cholesterol in the prediction of atherosclerotic disease: new perspectives based on the Framingham Heart Study. Ann Intern Med 1995; 90:85-91], the incidence and the severity of atherosclerosis are closely related to the cholesterol level in the blood.

It is therefore very important to keep blood cholesterol under control. Another important cardiovascular risk factor is represented by increased levels of triglycerides (above 150-200 mg/dl), especially if accompanied by a reduced HDL cholesterol level (< 40 mg/dl) or within the so-called "metabolic syndrome". It is therefore also important to lower the level of triglycerides in the blood. Dietary supplements may help to achieve that goal.

Another pathogenetic factor of atherosclerosis is peroxidation of LDL. In fact LDLs are transported in the blood without particular pathological problems. However, if they are peroxidized by ROS (Reactive Oxygen Species); they pass through the endothelium of the arteries and are captured and stored by macrophages. The latter, packed with peroxidized LDL, are transformed to so-called "foam cells", which represent the initial nucleus of atheromas of the intima, from which atherosclerotic plaques then form. Foam cell formation is considered one of the main drivers of atheromatous plaque formation and progression of atherosclerotic disease.

Antioxidants that inhibit the peroxidation of LDL may therefore be very useful for preventing atherosclerosis. This objective can be achieved with an appropriate choice of foods and with the support of suitable dietary supplements rich in antioxidants.

Suitable dietary supplements may therefore be advantageously employed for controlling the cholesterol level in the blood when this is slightly or moderately raised. Dietary supplements may be particularly useful for subjects with "intermediate" overall risk, or those having a probability between 10% and 20% of developing a cardiovascular disease in the next 10 years of life, but for whom therapy with cholesterol-lowering drugs is not yet justified.

There are at present various dietary supplements that can provide beneficial action and give a significant reduction of cardiovascular risk, especially if combined with a controlled diet and with healthy physical activity. There remains a need however for more effective, more efficient and safer compositions for improving the health or condition of the vascular system. In particular there is a need for such compositions based on natural ingredients as opposed to synthetic ingredients.

### Summary of the invention

The invention relates to a composition for oral administration having a beneficial effect on the cardiovascular system, comprising as active ingredients a mixture of monacolin K, lycopene and vitamin D3. Such a composition may further advantageously comprise at least one compound selected from the group consisting of berberine, coenzyme Q10, panax ginseng, flavenoids, vitamin K2, folic acid, vitamin B, biotin or minerals.

The invention also relates to a composition as described above for use as a medicament, i.e. for use in the treatment or prevention of a disease or for reducing foam cell formation and progression. A particular preferred use of the composition according to the invention is in the treatment of heart disease, atherosclerose, cardiovascular disease, hypercholesterolaemia, hypertriglyceridaemia, and other diseases related to an increased uptake of oxLDL by foam cells.

### Detailed description of the invention

Atherosclerosis and the resulting plaque formation in the vessel wall is the underlying mechanism of cardiovascular disease. Atherosclerosis is a progressive inflammation driven process. It is believed that disturbed cholesterol levels, hypertension and elevated blood glucose levels not only contribute to disease progression, but are also driven by the same inflammatory processes that cause atherosclerosis.

We employed a model for measuring the oxLDL uptake by macrophages, a known and accepted model for determining foam cell formation, which in turn is an accepted measure for plaque formation as well as for the development of atherosclerosis and decreasing vascular health in general. Our experimental data are therefore relevant for determining the beneficial effects of several active ingredients on the cardiovascular system.

More in detail, we stimulated human human monocytic THP-1 cells [Tsuchiya S, Yamabe M, Yamaguchi Y, Kobayashi Y, Konno T, Tada K (August 1980). "Establishment and characterization of a human acute monocytic leukemia cell line (THP-1)". Int. J. Cancer 26 (2): 171-6. doi:10.1002/ijc.2910260208.] with phorbol 12-myristate 13-acetate (PMA) and contacted or pre-treated the cells with a number of active ingredients. After 24 hours of pre-treatment, the cells were contacted with or exposed to fluorescently labeled oxLDL and uptake of oxLDL was determined after 24 hours (see example 1).

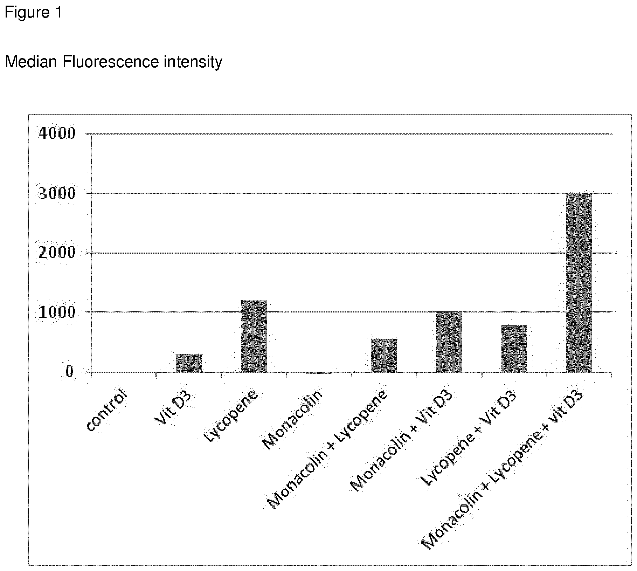

We found that addition of vitamin D3 and monacolin K had no effect on the uptake of oxLDL, whereas lycopene slightly inhibited the uptake of oxLDL (figure 1). Compositions comprising a combination of two of these three active ingredients did not alter the inhibitory effects significantly. Surprisingly, a composition comprising all 3 active ingredients exhibited a synergistic effect. When monacolin K, vitamin D3 and lycopene were combined in one composition, that composition gave rise to an inhibition of the uptake of oxLDL that was far greater than the sum of the effects of the active ingredients individually (example 1, figure 1).

Uptake of oxLDL by macrophages leads to the development of foam cells, which contribute to atherosclerotic plaque formation and progression. The invention therefore relates to a composition having a beneficial effect on the cardiovascular system, comprising as active ingredients a mixture of monacolin K, lycopene and vitamin D3. Such a composition is preferably for use in oral administration.

Whereas the individual active ingredients are known and have been described for various medicinal uses, a composition as described above is novel. Moreover, the composition according to the invention surprisingly showed a synergistic effect on the inhibition of oxLDL uptake of macrophages, thereby reducing the formation of foam cells. This has a beneficial effect on the cardiovascular system and reduces the chances of acute myocardial infarction and stroke, as well as atherosclerotic plaque formation. A composition according to the invention provides the additional advantage that it may contain less of each of the active ingredients than in a comparable composition that comprises each of the ingredients separately, whereas it achieves the same effect. This feature allows for the addition of other ingredients, since the number of ingredients that is contained in a composition is limited. This is attributable to the fact that the weight of a unit dose for oral administration may be approximately 1 gram in total. Above that weight, the unit dose becomes difficult to swallow, a unit dose of 5 grams is almost impossible to swallow. The fact that the three ingredients mentioned above synergistically improve their effect, allows for further additional active ingredients to be contained in the same unit dose.

A composition according to the invention also has a strong anti-inflammatory effect. As such, a composition according to the invention may significantly contribute to the prevention of cardiovascular disease (CVD), by lowering the CVD risk factors and reducing inflammation. Therefore, the composition according to the invention has the potential to reduce disease progression by early intervention, delaying or preventing the need for medical treatment of hypercholesterolemia, hypertension and type 2 diabetes.

The individual active ingredients of the composition according to the invention are herein described in detail.

"A", "an", and "the" as used herein refers to both singular and plural referents unless the context clearly dictates otherwise. By way of example, "an ingredient" may refer to one or more than one ingredient.

"About" as used herein referring to a measurable value such as a parameter, an amount, a temporal duration, and the like, is meant to encompass variations of +/-20% or less, preferably +/-10% or less, more preferably +/-5% or less, even more preferably +/-1% or less, and still more preferably +/-0.1 % or less of and from the specified value, in so far such variations are appropriate to perform in the disclosed invention. However, it is to be understood that the value to which the modifier "about" refers is itself also specifically disclosed.

The recitation of numerical ranges by endpoints includes all numbers and fractions subsumed within that range, as well as the recited endpoints.

The expression "percentage" "%" or "% by weight" (weight percent), here and throughout the description unless otherwise defined, refers to the relative weight of the respective component based on the overall weight of the formulation.

Monacolin K is a major constituent of red yeast rice [references 2 - 13]. Red Yeast Rice is rice that has been fermented with the yeast Monascus Purpureus. Its constituent called monacolin K has similar properties as the commonly known statins in cholesterol lowering medicine. Monacolin K is also known as the statin Lovastatin or Mevacor, which is marketed by Merck as cholesterol lowering medicine. It lowers the body's cholesterol production by inhibiting HMG-CoA reductase in the liver.

Over 100 clinical trials have been performed that analyzed the cholesterol lowering properties and preventive efficacy of Red Yeast Rice. These studies all show that daily consumption of Red Yeast Rice has similar cholesterol lowering properties as medicinal statins, but with fewer side effects. A meta-analysis of 9625 patients in 93 randomized trials involving 3 different commercial variants of Red Yeast Rice has summarized this large experience (4). In addition, a standardized Red Yeast Rice extract called Xuezhikang was evaluated in a large, randomized, placebo-controlled clinical trial, the China Coronary Secondary Prevention Study (CCSPS). With a duration of 4.5 years the CCSPS enrolled 4870 participants (3986 men, 884 women) with a previous myocardial infarction and showed significant reduction in the primary endpoint, nonfatal myocardial infarction or death from coronary or cardiac causes. The secondary endpoints, including total mortality from cardiovascular disease, total all-cause mortality, need for coronary revascularization procedure, and change in lipid levels, were also significantly reduced, with the exception of fatal myocardial infarction. The number needed to treat (NNT) to prevent a primary end point over the 4.5-year duration of the trial is 21, which favorably compares with the NNT range (19-56) observed in previous secondary prevention studies of statins.

Scientific evidence shows that daily intake of Red Yeast Rice containing 10mg monacolin K results in LDL cholesterol lowering of 7-25%, relative increase of HDL up to 17% and lowering of triglyceride blood levels by 10-44%. By lowering these risk factors the cardiovascular risk was reduced by 30-50% (2-13).

Besides cholesterol lowering, statins also have anti-inflammatory properties. This is a major constituent of the preventive effects of statins on cardiovascular disease. Several meta-analyses together containing over 30 clinical studies show that statin use reduces the blood levels of a number of well-known inflammatory markers (70, 71). In addition, statins have been shown to have immunomodulator effects in immune diseases like rheumatoid arthritis (72). Meta-analyses of over 20 studies show that stains help to prevent infections and infection induced mortality (73, 74). Recent studies show that daily statin use in low-risk individuals results in a significant reduction of cardiovascular risk and cardiovascular related mortality (75).

Besides natural statins, Red Yeast Rice also contains a series of compounds with beneficiary effects on cardiovascular disease, such as polyunsaturated fatty acids, ergosterol, amino acids, flavonoids, alkaloids, tannins and different trace elements and minerals (76).

The safety of Red yeast Rice consumption has been elaborately shown in recent clinical trials and meta-analyses. In the large meta-analysis that evaluated 93 randomized clinical trials (including 9625 participants), Red Yeast Rice preparations showed cholesterol-lowering effects similar to those of low-dose statins with no relevant adverse effects (4). Red Yeast Rice extract with a 0.2 % monacolin K content is well tolerated, also in statin intolerant subjects, at the dosage of 0.6, 1.2, or 2.4 g twice daily, (90, 91). A study that specifically investigated the side effects of 1.8 g/day Red Yeast Rice in patients that did not tolerate standard statin therapy, showed that 93 % of subjects with a history of statin-associated myalgia were able to tolerate 3.6 grams (equivalent to 6mg lovastatin) of Red Yeast Rice daily for 24 weeks without a recurrence of myalgia. Furthermore, in this study Red Yeast Rice did not increase the mean brief pain inventory (BPI) score, which represents reported average pain over the past month on a scale of 1-10. There also was no significant difference in creatine phosphokinase, aspartate aminotrasferase, or alanine aminotransferase levels between treatment and control groups. In this study 56% of subjects achieved target cholesterol levels (91). A one year study of a dietary supplement containing Berberine 500 mg, Policosanol 10 mg, Red Yeast Rice 200 mg, Folic acid 0.2 mg, Coenzyme Q10 2.0 mg, and Astaxanthin 0.5 mg in 80 elderly hypercholesterolemic patients who were previously statin-intolerant, showed that acceptable plasma LDL-C levels can be achieved without significant side-effects or safety issues. In addition, no significant differences in creatine phosphokinase, aspartate aminotrasferase, or alanine aminotransferase levels between treatment and control groups were reported (19). Interestingly however, there are also a number of case reports in the literature of muscle myopathy and liver damage resulting from red yeast rice usage (82-85, 92-94).

In conclusion, at comparable doses, the monacolin K efficacy and safety profile is similar to that of statins. In a composition according to the invention, monacolin K may preferably be present in the range of 2 - 40 mg per unit dose.

A unit dose is defined herein as a single-unit container so designed that the contents are administered to the patient as a single dose, direct from the container. Preferably, the unit dose comprises the daily dose so that a patient only has to take a single unit per day. A unit dose may comprise a pill, sachet, capsule, bulk powder container, tablet or equivalent suited for oral administration to a subject, preferably a human.

In a preferred composition according to the invention a low dose of citrinin free Red Yeast Rice extract containing 10 mg monacolin K per dose unit is used. This is 2 times less than the commonly used 20 mg starting dose, and 40-100 mg treatment dose of Lovastatin, thereby reducing the risk of side-effects.

Lycopene (from the New Latin word lycopersicum for the tomato species name) is a bright red carotenoid pigment and phytochemical found in tomatoes and other red fruits and vegetables, such as red carrots, red bell peppers, watermelons, and papayas (but not strawberries or cherries). Lycopene is not an essential nutrient for humans, but is commonly found in the diet, mainly from dishes prepared from tomatoes. Lycopene has been found to possess antioxidant and anti-proliferative properties in animal and laboratory studies, although activity in humans is not yet adequately determined. Lycopene is currently marketed as a protective agent against prostate cancer. However, conclusive evidence for this effect is lacking. Lycopene does have positive effects on cholesterol levels and blood pressure, potentially reducing cardiovascular disease risk.

To our knowledge, no case control studies that evaluated Lycopene supplementation and the risk of cardiovascular disease are available. There are, however, several studies that associated high dietary lycopene consumption with cardiovascular disease risk. In a major European study, men who ate large amounts of lycopene-rich foods were 50% less likely to have a heart attack than men who consumed little lycopene. Nonsmokers experienced the most benefit (113). Some epidemiological research also suggests that women with higher serum lycopene levels have a reduced risk of developing heart disease (114). However, conflicting evidence exists. A seven-year prospective cohort study of 39,876 middle-aged and older women assessed whether intake of lycopene or tomato-based foods is associated with cardiovascular disease prevention. Results from the study showed no association between dietary lycopene intake and the risk of heart attack, stroke, and other cardiovascular events in women (115). The dietary intake in these studies is significantly lower than what is achieved by lycopene supplementation. In addition high dietary lycopene intake often is associated with other dietary differences, making it hard to draw definite conclusions.

In a meta-analysis of 12 studies that investigated the effect of lycopene consumption on serum lipids, and four studies that examined its effect on blood pressure including data of 694 participants, revealed a significant cholesterol-lowering effect of lycopene for total serum cholesterol and LDL cholesterol by 10%, while HDL levels remained unchanged. This result was only achieved in the subgroup of trials using lycopene dosages of ≥25mg daily, whereas subgroup meta-analysis of trials using lower lycopene dosages was not significant (28). Another study showed that lycopene intervention reduces inflammation and improves HDL functionality in moderately overweight middle-aged individuals (29). The protective effect of Lycopene can also be deducted from a study in 1031 Finnish men aged 46-65 years of the Kuopio Ischemic Heart Disease Risk Factor (KIHD) cohort that showed that low blood levels of lycopene and/or β-carotene are associated with increased risk of sudden cardiac death (25-27). A recent study showed that lycopene inhibits 3-hydroxy-3-methylglutaryl coenzyme A (HmG-CoA) reductase in white blood cells, specifically, monocytes and macrophages, reducing or preventing foam cell formation. Foam cell formation is an important aspect of cardiovascular disease development. This inhibitory effect on HMG-CoA reductase was confirmed in another study (116). This mechanism of action is similar to that of statins (117).

A relatively small study in 25 subjects indicated that consuming Lycopene rich tomato products with a high-fat meal attenuates postprandial lipemia-induced oxidative stress and associated inflammatory response. The relevance of oxidized LDL and inflammation to vascular injury suggests a potentially important protective role of lycopene in reducing cardiovascular disease risk (118).

A meta-analysis of a limited number of 4 studies to the effect of lycopene on systolic blood pressure suggested a significant blood pressure reducing effect of 5.60 mmHg. Blood pressure reduction was more pronounced (9,35 mmHg) when a subgroup of trials with high blood pressure at baseline (SBP ≥ 140 mm Hg) was analyzed (28).

In one of the largest, most comprehensive studies evaluating prostate cancer risk and lycopene, a cohort of more than 47,800 men completed a 131-item questionnaire in 1986 and every few years after. Men with higher estimated lycopene content in their diets had a lower risk of developing prostate cancer. A risk reduction of 35% was observed for men who consumed ten or more servings of tomato products per week compared to those with fewer than 1.5 servings per week. Interestingly, tomato sauce accounted for the strongest reduction in risk for any one specific food item (119). But a 2004 meta-analysis of 11 studies found that while tomato products play a role in preventing prostate cancer, their role is modest and requires high amounts of tomato intake (120). A recent Cochrane meta-analysis on the protective effects against prostate cancer remains inconclusive (121-123). Three randomized controlled clinical trials, with a total of 154 participants were included in this review. None of the studies reported data on prostate cancer mortality. All of the included studies differed with respect to design, participants included and allocation of lycopene. This clinical heterogeneity and the reported 'high' risk of bias limits the value of the meta-analyses. Meta-analysis indicated no statistical difference in PSA levels between lycopene and placebo. PSA is a biomarker for prostate cancer. Only one study reported a decreased incidence of prostate cancer, 10% in the lycopene group versus 30% in control group. Another meta-analysis showed no correlation between lycopene consumption and breast cancer risk (20). The role of lycopene supplementation is not yet clear. Preliminary evidence shows that in men with existing high-grade prostate intraepithelial neoplasia (pre-malignant cellular changes seen in biopsy specimens), taking 4 mg of lycopene twice daily may delay or prevent progression to prostate cancer (125). However, preliminary evidence indicates lycopene may worsen established prostate cancer by increasing metastasis without having any effect on cancer cell proliferation (126). In all there is insufficient evidence to either support, or refute the use of lycopene for the prevention of prostate cancer. Similarly, there is no robust evidence from clinical trials to identify the impact of lycopene consumption upon the incidence of prostate cancer, prostate symptoms, PSA levels or adverse events.

In a 2005 prospective cohort study of dietary lycopene and its food sources evaluated in 39,876 women, neither higher dietary nor plasma lycopene levels were associated with a reduced risk of breast cancer in middle-aged and older women (127). In the 2007 evidence-based review by the FDA, data showed limited evidence to support an association between lycopene intake or tomato consumption with a reduced risk of breast cancer (128). Blood levels of lycopene and other carotenoids do, however, show significant associations with reduce breast cancer risk (129). However, some positive relationships between lycopene and reduced risk of breast cancer were shown in the Women's Health Initiative studies and in the Shanghai Women's Health Study when subgroups of carotenoids were examined, but these effects have not been examined individually in clinical trials (130, 131). In a 2007 evidence-based review, the U.S. Food and Drug Administration (FDA) found only limited evidence to support an association between lycopene intake or tomato consumption with a reduced risk of gastric cancer (128).

Lycopene, extracted from tomatoes or other natural sources is nontoxic, is generally considered safe and is authorised as a food colouring agent within the EU (E160d) (Directive 94/36/EC) and the US (CDR 21 73.295). It is a common food ingredient. An overview of average dietary intakes of lycopene from foods in different populations was presented in previous EFSA evaluations. Regular intakes of lycopene from natural dietary sources are estimated to be on average between 0.5 and 5 mg/day, with high exposures up to about 8 mg/day. High consumption of fruits and vegetables, especially tomato products, may result in occasional intakes of 20 mg/day or more. The acceptable daily intake (ADI) established by the Scientific Panel on food additives, flavourings, processing aids and materials in contact with food (AFC Panel) is 0.5 mg/kg/day for lycopene from all sources (EFSA, 2008). This translates to a daily intake of 30 mg Lycopene for someone weighing 60 kg. The dose of 25 mg in a preferred composition according to the invention lies well within this ADI.

There are no known serious side effects, but the safety of long-term intake of high doses of lycopene supplements has not been thoroughly studied. Review of available scientific literature finds tomatoes, tomato-based products, and lycopene supplements generally well tolerated. Daily supplements containing 30 mg of lycopene have been used safely for up to 8 weeks However, rare reports of diarrhea, nausea, stomach pain or cramps, gas, vomiting, and loss of appetite have been reported by some individuals who took a lycopene-rich tomato supplement of 15 milligrams twice a day. There are no known interactions between lycopene and existing drugs.

One important, but still unclear, safety concern is the fact that preliminary evidence indicates lycopene may worsen established prostate cancer by increasing metastasis without having an effect on cancer cell proliferation. Therefore, men with established prostate cancer should avoid using lycopene until more is known (126).

A composition according to the invention may contain between 1 and 100 mg lycopene per unit dose, such as between 5 and 50 mg. Preferred is a dose of 25 mg per unit dose.

Recent research established that a wide range of health benefits that can be achieved by vitamin D supplementation. The best known are the muscular and the skeletal benefits to prevent Rickets. The Mayo Clinic provides a very good overview of existing scientific evidence for the health benefits of vitamin D supplements at http://www.mayoclinic.com/health/vitamin-d/NS_patient-vitamind/DSECTION=evidence the content of which is incorporated by reference herein.

The current International Osteoporosis Guidelines determine vitamin D insufficiency as vitamin D (25-OHD) blood levels <50 nmol/L, while vitamin D deficiency is determined as 25-OHD blood levels <25 nmol/L. Based on these Guidelines the majority of people (aged >45 years) has insufficient or sub-optimal vitamin D levels (187-189). The summary below will focus on the benefits of vitamin D supplementation in cardiovascular disease prevention.

Vitamin D deficiency or sub-optimal vitamin D levels are related with increased cardiovascular risk. Vitamin D deficiency has been linked with hypertension, immune function, myocardial infarction, stroke, and other cardiovascular-related diseases including atherosclerosis and endothelial dysfunction (45-49). A large prospective, case-controlled study of 18,000 men showed a significant correlation between low vitamin D levels and an increased risk for myocardial infarction (50). A Meta-analysis of 24 studies containing a total of 6123 cardiovascular disease cases in 65 994 participants, also showed that lower vitamin D levels are linearly associated with increased cardiovascular risk and increased risk of cardiovascular disease-related mortality (51-55).

A meta-analysis of 17 prospective cohort studies and randomized trials found moderate to high doses of vitamin D supplementation may decrease the risk for cardiovascular disease. However, the amount of studies are limited and the available data is not very consistent (190). Only one study in the general population showed a very clear decrease in cardiovascular disease after vitamin D supplementation (56). In another trial of 148 women supplementation with vitamin D and calcium resulted in a significant increase in vitamin D blood levels by 72% and significant decreases in systolic blood pressure and heart rate compared with calcium supplementation alone (57). However, a different meta-analysis of 51 trials examining the effects of vitamin D supplements on cardiovascular outcomes found that although some trials showed benefits of vitamin D supplementation on risk factors, the overall trial data available could not demonstrate a consistent and statistically significant reduction in mortality or cardiovascular risk after vitamin D supplementation (191, 192). The authors do suggest a positive effect of vitamin D supplements as was seen in a different meta-analysis (55). They blame the fact that many of the included studies were not designed to evaluate cardiovascular outcomes.

Recent studies show low vitamin D levels are associated with hypertension and left ventricular hypertrophy. Supplementation with vitamin D provides relieve for left ventricular atrophy (58, 59). Two meta-analyses have looked at the effect of vitamin D on blood pressure levels. These meta-analyses did not demonstrate a significant effect of vitamin D on blood pressure (193, 194). However, included trials were small and significant heterogeneity was observed in both meta-analyses. Better designed trials are needed to thoroughly investigate the benefits of vitamin D supplementation in lowering blood pressure.

Other studies showed an association between low vitamin D levels and endothelial dysfunction and arterial stiffness (49). Supplementation of vitamin D in patients with low vitamin D levels showed significant improvement in arterial stiffness when compared with placebo. Furthermore studies have also shown an association between vitamin D supplementation and a decrease in pulse wave velocity and arterial stiffness (60, 61) and an inverse association between vitamin D levels and subclinical atherosclerosis as measured by carotid intimal-media thickness (62). These studies do suggest a beneficial effect of vitamin D supplementation on cardiovascular risk.

Studies show a chronic deficiency in vitamin D among people aged over 45, this is mostly acclaimed to the lack of exposure to sunlight and reduced vitamin D production. In addition, research shows that more and more age related disorders are associated to sub-optimal vitamin D levels. Although conclusive evidence of the health benefits of vitamin D supplements on cardiovascular disease risk is still lacking, many studies suggest positive effects. Despite the limited evidence for cardiovascular disease prevention, the recommendation of vitamin D supplements is justified by the wide spread insufficiency and the scientifically proven muscular and the skeletal health benefits. In conclusion, vitamin D supplements are recommended for people >45 years of age.

A composition according to the invention preferably comprises between 1 and 100 microgram of vitamin D, more preferably between 5 and 50, such as 25 microgram per unit dose.

In addition to the above described active ingredients, a composition according to the invention may also contain a number of additional ingredients. The invention therefore also relates to a composition as described herein further comprising an active ingredient selected from the group consisting of berberin, coenzyme Q10, panax Ginseng, flavenoids, vitamin K2, folic acid, vitamin B, biotin and minerals.

Berberine is a strongly yellow colored quaternary ammonium salt that is found in Berberis plants (e.g. Oregon grape, barberry, and tree turmeric), goldenseal and Chinese goldthread. Berberine is usually found in the roots, rhizomes, stems, and bark. Many studies have shown Berberine has various beneficial effects on the cardiovascular system, such as cholesterol lowering, significant anti-inflammatory activities and reduction of blood glucose levels similar to anti-diabetic medication (Metformin). This was confirmed in a recent meta-analysis of 14 randomized trials involving 1068 participants that concluded that based on the existing evidence reviewed, Berberine has beneficial effects on blood glucose control in the treatment of type 2 diabetic patients and exhibits efficacy comparable with that of conventional oral hypoglycaemics. According to this analysis the antidyslipidemic effect of berberine is highly promising but needs further confirmation. Additionally, it has no serious adverse effects except for a mild to moderate gastrointestinal discomfort (95).

Clinical studies reveal that Berberine reduces total cholesterol, LDL-C and triglyceride levels by 15-18%, 17-21% and 21-36% respectively (14-17). The lipid lowering effect of Berberine is different from and complementary to statins as was shown in animal studies (18). Berberine leads to an increase in LDL receptor by reducing its breakdown, leading to increased uptake of LDL from the blood by the liver. Several successful clinical studies involving in total 2025 participants have been performed to determine the protective effect of a combination of Berberine, Red Yeast Rice and Policosanol to cardiovascular disease risk (19-22). These studies showed significantly lowering of total cholesterol (17-20%), LDL-C (23-31%), triglycerides (13%) and insulin resistance (10%), and a relative increase in HDL levels in patients with elevated cholesterol levels. This result was associated with improved endothelial function (20, 23).

Berberine increases insulin receptor (InsR) expression and improves insulin sensitivity (24). A study in 79 participants with elevated blood glucose levels, showed that Berberine significantly lowered fasting blood glucose and lowered levels of the diabetes marker haemoglobin A(1c) (HBA1c) and insulin. In this study the blood glucose- and HBA1c-lowering efficacies of Berberine were similar to those of the well-known diabetes medication Metformin. Berberine also lowered fasting blood glucose effectively in 35 chronic hepatitis B and hepatitis C patients with type 2 diabetes or impaired fasting glucose. Liver function was improved greatly in these patients by showing reduction of liver enzymes (16). This was confirmed in another study in which HBA1c decreased from 8.1% to 7.3%. Fasting plasma insulin and insulin resistance index were reduced by 28.1% and 44.7% (P<.001), respectively. In addition, the glucose disposal rate was increased and thus the insulin resistance decreased after Berberine treatment (14, 15).

In a recent study, Berberine was revealed to markedly reduce the inflammatory cell adhesion to endothelial cells that is induced by oxidized LDL. This inhibitory mechanism of Berberine was associated with suppression of adhesion molecule expression (VCAM-1 and ICAM-1) on endothelial cells. These results indicate that Berberine has a protective role in the early stages of atherosclerosis.

Congestive heart failure is a condition in which the heart's function as a pump is inadequate to deliver oxygen rich blood to the body. A study from 1988 showed in a small number of 12 patients with refractory congestive heart failure that continuous intravenous injection of a high dose Berberine resulted in strongly increased heart function and reduced vascular resistance (96). These results were confirmed in a more recent study from 2003. This study showed Berberine improved quality of life and heart function and decreased mortality in 156 patients with congestive heart failure (97).

The supposed mechanism of action of Berberine is the increased expression of the liver receptors for LDL (LDLr) and insulin (InsR). Besides its up-regulating effect on LDLr and InsR, berberine could also reduce triglycerides by AMP kinase activation and MAPK/ERK pathway blocking (14). The pharmacological actions of Berberine are summarized in a an elaborate review by Cicero and Ertek (98). Due to its peculiar mechanism of action not directly involving the HMGCoA reductase, berberine has been observed to increase the cholesterol-lowering action of both simvastatin and monacolin Kes (17).

Berberine is not considered toxic at the doses used in clinical situations, nor has it been shown to be cytotoxic or mutagenic. The metabolism of Berberine by liver enzymes and the effect of the metabolites on LDLr and InsR expression was characterized by Li et al. (99). In a rat noncompartmental model, Berberine is metabolized by p450 enzyme system in liver, with phase I demethylation and phase II glucuronidation. Berberine has four main metabolites identified in rats: berberrubine, thalifendine, demethyleneberberine, and jatrorrhizine, all of which have glucuronide conjugates (99). The LD50 of berberine HCL is >29586 mg/kg in mice and >15 g/kg in rats (100). In clinical studies performed thus far standard doses below 2 g/day of berberine are well tolerated and adverse reactions are rare and mild.

In the meta analysis of Chinese studies to the efficacy of Berberine by Dong et al. (95), eleven of fourteen trials reported outcomes for adverse effects whereas the rest reported no adverse effects during the Berberine treatment. Three of these reported the incidence of abdominal discomfort, but did not report the group in which abdominal discomfort occurred. Five trials appointed the abdominal discomfort to the Berberine intervention group. In the trial of Cao (101), there were seven incidences of abdominal discomfort like nausea, abdominal distension, and diarrhea. The symptoms were relieved by taking the Berberine postprandial instead of on an empty stomach as initially prescribed. Li and Liu (102) reported a few patients who developed a mild diarrhea caused by the intake of Berberine. In the trial of Wang (103), one incidence of constipation occurred and was relieved after reducing the dose of Berberine to 0.2 g three times a day instead of 0.5 g thrice daily. Zhang et al. (15) also reported mild-to moderate constipation in five participants who took Berberine. Constipation in three participants in the Berberine group was relieved without dose reduction, and two patients with mild constipation reduced Berberine dose to 0.25 g twice daily, resolving the adverse effects. In the trial of Ye (104), the tolerable mild constipation occurred. No severe hypoglycemia was observed in all the included trials in the meta-analysis. In all trials performed thus far no serious adverse events were observed.

On the contrary, high doses equivalent to > 2 g/day of Berberine in isolated cases have been associated with dyspnea, flu-like symptoms and cardiac damage. However, these adverse effects have not been substantiated by clinical trial data. Nevertheless, the most frequent and most studied side effects are those on the gastrointestinal system. In fact, high dose Berberine and derivatives can produce gastric lesions. By using sorbitol and breath hydrogen tests to measure small intestinal transit time, it has been shown that Berberine delays small intestinal transit time. This may account for part of its gastrointestinal and antidiarrheal side effect (105, 106). Another important side effect is that Berberine displaces bilirubin from albumin. Therefore high doses of Berberine should be avoided in jaundiced infants and pregnant woman, as high bilirubin levels can stimulate uterus contraction (107, 108). A composition according to the invention may therefore advantageously contain between 100 and 500 mg of berberine, such as 200 - 400 mg per unit dose, such as 300 mg berberine per unit dose.

The main safety issue of Berberine involves the risk of some pharmacological interaction. Berberine can bind Heparin and therefore has potential interactions with anticoagulant treatment using heparin (109). Berberine can also markedly increase blood levels of cyclosporine A, due to CYP3A4 and P-glycoprotein inhibition in the liver and gut wall. In addition, the increase in gastric emptying time causes increased cyclosporine A bioavailability. This is not a safety issue in healthy individuals, but in renal transplant recipients taking cyclosporine 3 mg/kg twice daily, the coadministration of berberine (0.2 g/day for three times a day for three months) increases the mean cyclosporine A Area under the Curve (AUC) by 34.5% and its mean half-life by 2.7 h (110). Interactions with other drugs that are metabolized by CYP3A4, such as warfarin, lovastatin (Mevacor), clarithromycin (Biaxin), indinavir (Crixivan), sildenafil (Viagra) and triazolam (Halcion) are also expected, but not substantiated by clinical evidence. Although the main mechanism of pharmacological interaction of Berberine involves CYP3A4 and intestinal P-glycoprotein, it was also found to inhibit CYP1A1 in vitro. Therefore Berberine potentially interacts with the relatively rare drugs that are metabolized by this cytochrome isoform (111). Some environmental contaminants such as aryl-hydrocarbons are also metabolized by CYP1A1 and the inhibitory effect of Berberine on CYP1A1 on these compounds has yet to be elucidated.

Finally, Berberine could interact with mitotic inhibitors used in cancer chemotherapy, like Paclitaxel. It was observed that a 24-h Berberine treatment up-regulated the multidrug-resistant transporter (pgp-170) expression in two oral, gastric and colon cancer cell lines. Decreased retention of rhodamine 123 was observed in Berberine-treated cells as compared to vehicle control. Pretreatment of cells with Berberine for 24 h prior to Paclitaxel treatment blocked the Paclitaxel-induced cell cycle responses and morphological changes, resulting in increased viability as compared to that of Paclitaxel-treated cells. These results suggest that Berberine modulates the expression and function of pgp-170 leading to a reduced response to Paclitaxel in digestive track cancer cells (112). Again, no clinical report of a significant pharmacological interaction is yet available.

Overall there are a large number of recent clinical trials supporting the safe use of this nutraceutical up to one year, especially when used at a lipid-lowering dosage < 2 g/day.

Coenzyme Q10 (CoQ10) is found in high concentrations in the mitochondria (power house) of every cell, it is involved in the energy metabolism (electron transport) of cells. As an electron carrier, the CoQ10 molecule is continually going through an oxidation-reduction cycle. As it accepts electrons, it becomes reduced (Ubiquinol) and becomes oxidized again (Ubiquinone) as it gives up electrons. CoQ10 is a powerful antioxidant that prevents oxidative stress caused by our body's energy metabolism. Because nearly all cellular functions depend on an adequate energy supply, CoQ10 deficiency affects all cells, particularly those with high energy consumption, such as the myocardium, neurons and immune cells.

CoQ10 can be synthesised by the body and therefore there is no need for CoQ10 in diets of healthy individuals. There are, however, a number of reported health benefits that can be achieved in populations with either hypertension, heart failure, dementia or people that take statins. Below the specific cardiovascular disease related health benefits of CoQ10 will be summarized.

There is no scientific evidence of a cholesterol lowering effect of coenzyme Q10.

Two meta-analyses one consisting of 3 clinical trials containing a total of 96 participants and one of 12 trials and 362 patients showed that treatment with CoQ10 in subjects with high blood pressure resulted in mean decreases in 11-17 mmHg in systolic and 7-10 mmHg in diastolic blood pressure without significant side effects (30, 31). A third meta-analysis concluded that favourable effects of CoQ10 on heart function (ejection fraction, exercise tolerance, cardiac output, and stroke volume) have been demonstrated in the literature, but further research is needed to recommend CoQ10 as medical therapy (32).

Endothelial dysfunction is one of the main causes of hypertension and has a role in atherosclerotic plaque development. A recent meta-analysis of 5 clinical trials with 194 patients concluded that CoQ10 supplementation is associated with significant improvement in endothelial function (33).

Statin therapy effectively reduces the body's cholesterol production by inhibiting Hydroxymethylglutaryl coenzyme A (HMG-CoA) reductase. However this enzyme is also responsible for the production of CoQ10. Studies have shown that CoQ10 levels are significantly reduced upon statin treatment (132-137). More and more studies demonstrate that CoQ10 supplementation can help to reduce side effects of statin treatment, most importantly myalgia and myopathy (muscle aches, rhabdomyalysis). Several studies have demonstrated reduced side effects of statin treatment after CoQ10 supplementation (34-37). However, some studies report no reduction of myalgia upon CoQ10 supplementation alongside statin treatment (138, 139). It must be noted that in these studies either the levels of myalgia were very low or the bioavailability of the supplement was not tested by measuring coenzyme Q10 blood levels. Thus far, no large clinical trials have confirmed this theory.

Patent EP0383432B1 describes the benefits of CoQ10 supplementation alongside statin treatment. The pharmaceutical use of CoQ10 to reduce statin induced myopathy is described therein.

A recent meta-analysis, consisting of 13 randomized controlled trials of in total 395 participants selected from of 120 potentially relevant studies, suggests that supplementation with CoQ10 may be of benefit in patients with congestive heart failure. However, our findings also suggest that the benefit may be limited to patients with less severe stages of CHF, such as patients with an ejection fraction ≥30% or those with an New York Heart Association (NYHA) functional classification class of II or III. Because of the small number of studies and patients included in this meta-analysis, the results should be interpreted with caution. Although there is a possible benefit of supplementation with CoQ10, additional larger studies are warranted and should examine whether there is an effect when this supplementation is added to the current standard of therapy for CHF or whether there is a dose-response effect between the stage of CHF at baseline and the dose of CoQ10 required for an improvement to be seen (140).

CoQ10 undergoes biotransformation in the liver and is eliminated primarily through the biliary tract, so it can accumulate in patients with hepatic impairment or bile duct obstruction (141). There are few reported serious adverse effects of CoQ10. Side effects are typically mild and brief. Taking 100 mg a day or more of CoQ10 has caused mild insomnia in some people. Research has detected elevated levels of liver enzymes in people taking doses of 300 mg per day for long periods of time. Liver toxicity has not been reported. Other reported side effects include rashes, skin itching, nausea, vomiting, stomach upset, diarrhea, loss of appetite, upper abdominal pain, dizziness, sensitivity to light, irritability, headache, heartburn, fatigue or flu-like symptoms (142).

The acceptable daily intake (ADI) is 12mg/kg/day, calculated from the no-observed-adverse-effect level (NOAEL) of 1200 mg/kg/day derived from a 52-week chronic toxicity study in rats, this corresponds with 720 mg/day for a person weighing 60 kg. Risk assessment for CoQ10 based on various clinical trial data indicates that the observed safety level (OSL) for CoQ10 is 1200 mg/day/person.

CoQ10 does have some interactions with existing drugs. Since CoQ10 is able to decrease blood pressure caution is advised for individuals with low blood pressure or taking blood pressure medications. In addition, CoQ10 can reduce the body's response to warfarin (Coumadin) by acting as a vitamin K substitute, leading to altered dosing requirements for warfarin (143). Coenzyme Q10 may improve beta-cell function and enhance insulin sensitivity, which may reduce insulin requirements for diabetic patients (144, 145). One study indicated that Thyroid hormone levels are associated to CoQ10 levels and might be altered after CoQ10 supplementation (146).

A composition according to the invention may therefore advantageously contain between 1 and 200 mg of coenzyme Q10, such as 100 mg coenzyme Q10 per unit dose.

Ginseng is a well-known medical herb with a long list of acclaimed health benefits. Ginsenosides, known as ginseng saponins, are the major components of ginseng. Ginseng also contains several valuable nonsaponin components, including essential oils, antioxidants, polyacetylenic alcohols, peptides, amino acids, polysaccharides, and vitamins. A very elaborate overview of ginseng's medical applications is given by Jae Joon Wee et al. (147).

One of the most important acclaimed health effects is protection of neurons, which leads to improved learning and memory and prevention of Alzheimer's disease. However these effects have not yet been confirmed in human trials. In case-control studies, the chance of cancer of the lip, oral cavity and pharynx, larynx, lung, oesophagus, stomach, liver, pancreas, ovary, and colorectum were significantly reduced after using ginseng supplements (148, 149). In addition ginseng has been suggested to be effective in improving psychomotor function, cognitive functioning (150, 151), erectile function (152), and pulmonary disease (153). The following in depth information will be limited to ginseng's acclaimed health benefits for cardiovascular disease prevention.

Vasodilation (relaxing of the vessel wall), ischemic heart disease and angina pectoris (heart related chest pain).

Ginseng-based medicines and nitrates are commonly used in treating ischemic heart disease (IHD) and angina pectoris in China. Hundreds of randomized controlled trials reported in Chinese language claimed that ginseng-based medicines can relieve the symptoms of IHD by vasodilation of the coronary arteries. A systematic review containing eighteen randomized placebo controlled trials with 1549 participants investigating ginseng versus nitrates in treating IHD, demonstrated evidence that ginseng is more effective than nitrates for treating angina pectoris (38). These are highly promising results as nitrates are an accepted treatment in western countries for angina pectoris. This effect is attributed to the vasodilator function of ginseng, which has been shown in several animal studies and two small clinical trials (40, 154). The vasodilator function might also help to reduce blood pressure, but very limited evidence is available and seemingly high doses of ginseng are needed (39-41).

A very recent randomized controlled trial to the antioxidant activity of ginseng showed strong antioxidant effects in humans (42). Plasma superoxide dismutase (SOD), plasma glutathione peroxidase (GPx) and catalase activity were significantly higher after 8-week ginseng treatment. Furthermore, the DNA tail length and tail moment, both measures for DNA damage by oxidative stress were significantly reduced. More importantly, plasma levels of oxidized LDL, a major constituent to cardiovascular disease development, were reduced as well. Unfortunately study duration was short and sample size limited, making it hard to translate the study outcome to the general population.

The anti-inflammatory effects of Ginseng appear to be limited to men. A recent epidemiological study in 9,947 subjects showed that taking ginseng supplements is associated with a reduction of the inflammation risk marker for cardiovascular disease HSCRP by 16% in men. While ginseng supplements had no effect in women (43). It is interesting to note that in a large 18 year long cohort study of 6282 subjects, Yi et al. reported an inverse association between ginseng use and total mortality, an association which was similarly limited to men (44).

Several human randomized controlled clinical trials investigating the effect of red ginseng on type 2 diabetes have been performed but remain inconclusive (155). Clinical studies with small sample sizes (10-16 patients) have reported that American ginseng lowers postprandial blood glucose in diabetic and non-diabetic patients (156, 157). Animal studies clearly show protective effects of ginseng and its components against insulin resistance and diabetes. However, confirmation in human trials is lacking (155). Therefore, more studies are required to confirm that ginseng administration decreases the dietary glycaemic index and is preventive of insulin resistance.

A recent study in postmenopausal women showed significant improvements (Kupperman index and menopause rating scale) and reduction of total cholesterol and LDL after taking ginseng (158). However, no changes in cholesterol or triglycerides were detected in a study in hypertensive patients (41).

The use of Panax ginseng is generally considered safe. No reports of serious adverse effects are available. A recent systematic review of the efficacy and safety of Panax Ginseng including 57 clinical trials reported the following side effects (159). Thirty of the 57 trials reported the presence or absence of adverse events: 16 reported some side effects, whereas 14 found no side effects during the trials. The most common side effect is trouble sleeping (insomnia). Less commonly, people experience menstrual problems, breast pain, increased heart rate, high or low blood pressure, headache, loss of appetite, diarrhea, itching, rash, dizziness, mood changes, vaginal bleeding, abdominal pain, dyspepsia, hot flash and epistaxis. Most are associated with higher doses of Panax Ginseng of 500 mg per day. The 27 other trials did not address the topic.

There is not enough scientific evidence to support the safe use of Panax Ginseng during pregnancy or breastfeeding. There are some indications that Panax Ginsing might interfere with blood coagulation, therefore use in combination with anticoagulants requires careful monitoring of hemostasis.

A composition according to the invention may therefore advantageously contain between 1 and 40 mg of ginseng, such as 20 mg per unit dose.

Flavonoids are the most important plant pigments responsible for flower and fruit coloration producing yellow or red/blue pigmentation. The flavonoid subgroup anthocyanidins (anthocyanins) for example are the components that give berries (Blue-, cran- and bill-berries) their specific dark blue color. Other fruits and vegetables with red or orange colors also contain anthocyanins (166). Proanthocyanidins are the principal substances in red wine that are linked to a reduced risk of coronary heart disease and to lower overall mortality (167). Proanthocyanidins, refer to a different class of flavonoids, called flavanols, in which occur PCOs (proanthocyanidin oligomers) or the more common dietary supplement ingredient OPCs (oligomeric proanthocyanidins) or in more complex form the well-known tannins.

Several mechanisms of action of flavonoids in general have been described (168). Strong anti-oxidant induction properties. Oxidative stress has been implicated in the development of a number of conditions including cancer, arthritic disorders and cardiovascular disease. Stabilization of collagen fibers and promotion of collagen biosynthesis and decreased permeability and fragility of capillaries has been attributed to flavonoids. Also, flavonoids have been held responsible for inhibition of blood clotting (platelet aggregation), reduction of inflammation by prevention of the release and synthesis of pro-inflammatory compounds (e.g. histamine, prostaglandins, and leukotrienes) and lower blood glucose levels

Oxidative stress caused by free radicals is one of the driving forces behind ageing and the development of cardiovascular disease, dementia and cancer. The importance and protective effect of daily intake of antioxidants is well known and has been scientifically proven. Interestingly, most of the antioxidant function of the human body is not derived from the consumption of dietary antioxidants. Dietary antioxidants undeniably contribute to the body's antioxidant function, but the most potent antioxidants are produced by the body itself. The increased antioxidant function of the human body after consumption of fruits and vegetables is mostly due to an induction of the body's own antioxidant production by the flavonoids in them (169). Recently researchers discovered that the metabolites derived from digestion of flavonoids are light toxic substances. Due to their toxicity these substances activate the natural antioxidants and defense of the human body, such as the very strong antioxidant uric acid. These are far more efficient than dietary antioxidants and therefore offer highly valuable protection against oxidative stress (170-172).

Flavonoids have emerged as potential candidates to protect against cardiovascular disease. Many recent human studies suggest that the consumption of flavonoid rich food (Strawberries, Blueberries, Red Wine) decreases the risk of cardiovascular disease mortality. Relatively low-dose anthocyanin interventions (mostly in the form of pomegranate juice) with patients clinically diagnosed with vascular diseases have been associated with significant reductions in heart ischemia (173), intima-media thickness (thickness of the arterial wall), blood pressure, LDL oxidation (174), lipid levels (175), and inflammatory status (176). This last study was a double-blind, placebo-controlled, parallel trial in forty-four patients who survived myocardial infraction and have received statin therapy for at least 6 months. Chokeberry flavonoid extract (Aronia melanocarpa E, containing 25% anthocyanins, 50% polymeric proanthocyanidins and 9% phenolic acids) supplementation for a period of 6 weeks significantly reduced serum 8-isoprostans by 38% and oxLDL levels by 29%, as well as the inflammatory markers hsCRP by 23% and MCP-1 levels by 29%. In addition, significant increase in the protective adiponectin levels and reduction in systolic and diastolic blood pressure by a mean average of 11 and 7.2 mmHg, respectively were found. A recent study by Karlsen et al. (177) showed significant improvement of plasma risk biomarkers after supplementation with anthocyanins. Several pro-inflammatory cytokines and chemokines were reduced in the plasma of healthy men and women after supplementation with anthocyanins (178, 179). In view of the fact that flavonoids reduce the severity of inflammation, regardless of statins, they are a highly interesting supplementation alongside statin treatment (176).

A large number of cohort studies assessed the association between flavonoid intake and cardiovascular disease incidence and mortality. A recent review of existing studies revealed that associations are present between flavonoid intake and cardiovascular disease risk and mortality, but that results are not consistent (180). A meta-analysis of 6 cohort studies revealed that coronary heart disease risk decreased significantly with increased flavonoid intake. A large 16-year follow-up study in 34,489 post-menopausal women without cardiovascular disease showed that the intake of 0.2mg/day of the flavonoid classes, flavones and anthocyanins was associated with a decreased risk of cardiovascular disease (181). In another very recent study, this protective effect was substantiated (182). In 1999, a total of 38,180 men and 60,289 women, with a mean age of 70 and 69 y respectively were included in the Cancer Prevention Study II Nutrition Cohort. During 7 years of follow-up, 1589 cardiovascular disease related deaths in men and 1182 cardiovascular disease related deaths in women occurred. Men and women with total flavonoid intakes in the top (compared with the bottom) quintile had an 18% lower risk of fatal cardiovascular disease. Five flavonoid classes-anthocyanidins, flavan-3-ols, flavones, flavonols, and proanthocyanidins-were individually associated with lower risk of fatal cardiovascular disease (183). Several other large cohort studies showed that daily moderate red wine consumption resulted in reduced CVD risk (184-186). These effects are often attributed to the antioxidant properties of flavonoids.

A composition according to the invention may therefore advantageously contain between 1 and 500 mg of flavonoids, such as 200 - 400 mg per unit dose, such as 300 mg flavonoids per unit dose.

Vitamin K2 (menachinone, menaquinone, MK-4, MK-7, MK-9) has an important role in bone mineralization, arterial calcification, calcification of the heart valves tissue repair and according to a recent study also in insulin response. This is confirmed by recent studies showing that anti-coagulant medication (coumarin derivates) which inhibit vitamin K function lead to an increase in osteoporosis and artery calcification (195-197).

Vitamin K2 helps to reduce calcification of the arteries and heart valves. Two large Dutch studies have investigated this phenomenon. A study in 4,807 elder men and women, published in 2004 in the Journal of Nutrition showed that in the group with a high dietary intake of vitamin K2 had 57% less myocardial infarctions, 52% less calcification of the aorta and 26% less overall mortality compared to the group with al low dietary intake of vitamin K2 (63). After 8 years and 480 cardiovascular events, the cohort study Prospect EPIC in 16,057 women aged 49-70, showed that each increase in dietary intake of vitamin K2 by 10 microgram resulted in a risk reduction of 9% (64). Several other well-designed clinical trials showed that vitamin K2 helps to reduce osteoporosis (64-69).

At Geneva's Vitafoods 2012, Dr. Cees Vermeer, Principal Investigator at VitaK laboratory at Maastricht University, has presented data showing significant benefits for improved bone strength and prevention of cardiovascular aging with daily supplementation of MenaQ7. In the study, 244 healthy postmenopausal women received for 3 years daily 180 µg K2 from MenaQ7 or a placebo. The clinical measurements included bone mineral density, bone strength, vascular characteristics by ultrasound and pulse-wave velocity (PWV), the latter evaluating age-related stiffening of blood vessels. MenaQ7 supplementation provided a statistically significant protection of the most vulnerable bone structures i.e. vertebrae and the hip. The MenaQ7 trial showed substantial benefits in preventing age-related stiffening of arteries resulting in increase of a measure for arterial stiffness (Pulse Wave Velocity) in the placebo group, but not in the MenaQ7-group. Most remarkably, MenaQ7 not only prevented stiffening, it also resulted in a statistically significant improvement of vascular elasticity both measured with ultrasound techniques and Pulse Wave Velocity. http://www.cisionwire.com/nattopharma-asa/r/menaq7--3-year-human-study-with-vitamin-k2-demonstrates-that-menaq7-significantly-improves-bone-str,c9262978

There are indications that vitamin K2 helps to improve insulin response. However, this is not yet substantiated by large clinical trials (198).

A composition according to the invention may therefore also advantageously contain between 20 and 300 microgram of vitamin K2, such as 180 microgram per unit dose.

A composition according to the invention may also advantageously contain between 50 and 400 microgram of folic acid or B vitamins, preferably each at about 200 micrograms per dose unit.

A composition according to the invention may also advantageously contain between 0,5 and 4 mg of biotin (vitamin H), preferably each at about 2 mg per dose unit.

A composition according to the invention may also advantageously contain minerals, such as Calcium, Magnesium, Selenium, Zinc, Iodine or Chromium.

The invention also relates to the medical use of the compositions as described herein. In other terms, the invention relates to a method of treating a disease by administering a composition as described herein to a subject in need of such a treatment.

More in particular, the invention relates to a composition as described herein for use in the treatment or prevention of a disease or for reducing foam cell formation and progression.

The invention also relates to a composition for use as described above wherein the disease is selected from the group consisting of heart disease, cardiovascular disease, hypercholesterolaemia, hypertriglyceridaemia, hyperlipidemia, type II diabetes, elevated blood pressure, hypertension, pre-diabetes, myocardial infarction, stroke, heart failure, chronic heart failure and congestive heart failure.

Table 1 lists the active ingredients of the compositions according to the invention together with their preferred daily dose and ranges for administration.

**Table 1 list of active ingredients**

| **Active ingredient** | **Biological effect** | **Amount per unit dose (range)** |
|---|---|---|
| *Monacolin K* | • Natural Statin (HMG CoA inhibitor) Monacolin K | 10 mg (2 - 40) |
| | • LDL cholesterol lowering of 7-25% | |
| | • Relative increase of HDL up to 17% | |
| | • Lowering of triglyceride blood levels by 10-44% | |
| Lycopene | • LDL cholesterol lowering of 10% | 25 mg (1 - 100) |
| | • Systolic blood pressure lowering by 5.6-9.5 mmHg | |
| Vitamin D3 | • Overall CVD risk reduction | 25 microgram (1 - 100) |
| | • Reduction left ventricular hypertrophy | |
| | • Reduction arterial stiffness | |
| Berberine | • Upregulation LDL-receptor | 300 mg (100 - 500) |
| | • LDL cholesterol lowering of 17-25% | |
| | • Lowering of triglyceride blood levels by 21-36% | |
| | • Blood glucose- and HBA1c-lowering efficacies similar to metformin and rosiglitazone. | |
| Coenzyme Q10 | • Systolic blood pressure lowering by 11-17mmHg | 100 mg (1 - 200) |
| | • Diastolic blood pressure lowering by 7-10 mmHg | |
| Panax Ginseng | • Vasodilation | 20 mg (1 - 40) |
| | • Anti-oxidant | |
| | • Immune function | |
| | • Lowering oxLDL serum levels | |
| Flavonoids | • Vasodilation | 200 mg (1 - 500) |
| | • Anti-oxidant | |
| | • Immune function | |
| | • Lowering oxLDL serum levels | |
| Vitamin K2 | • Reduction of arterial calcification | 180 microgram (20 - 300) |
| | • Reduction of osteoporosis | |
| Folic acid + B vitamins | • | 200 microgram (50 - 400) |
| Biotin | • | 2 mg (0,5 - 4) |
| Total weight of | • | Less than 700 mg |
| preferred embodiment | | |

### Legend to the figures

Figure 1: Bar diagram showing the property of vitamin D3, monacolin K and lycopene to inhibit the uptake of oxLDL by pma-stimulated human THP-1 cells, individually and in synergy with each other.

### Examples

### Example 1 Model system

THP-1 cells (P7) were plated in a 96-wells suspension culture plate at a density of 1*10^6 cells/ml whereby 1*10^5 cells were plated. Cells were cultured in IMDM phenol-red free medium (Invitrogen, Eugene, OR, USA) enriched for 10% fetal calf serum (FCS), 100 U/ml penicillin, 100 µg/ml streptomycin and 2 mM L-glutamin. Monocytes were differentiated with 50ng/ml phorbol 12-myristate 13-acetate (PMA) (Sigma Aldrich, St. Louis, MO USA) for 48h where after medium was replaced. After differentiation, macrophages were cultured in the presence or absence of different compounds for 24h (Table 2). After pre-treatment, cells were exposed to 10ug/ml Dil-labeled oxidized lipoprotein (oxLDL) (Biotrend Chemikalien GmbH, Cologne, Germany) for another 24h at 37°C to enable foam cell formation in the absence of presence of the different compounds (Figure 1). After this, cells were washed with PBS (-/-) (PAA, Pitscataway, NJ, USA) and detached by Lidocaine hydrochloride (4mg/ml) EDTA (10mM) solution (Sigma Aldrich, St. Louis, MO USA)/ (Invitrogen, Eugene, OR, USA). Cells were washed two more times with FACS buffer (PBS containing 0,5% BSA and 0.2mM EDTA) and transferred to a 96 U-shaped plate for FACS analysis. Analysis was performed on the FACS Canto II HTS and 1,1'-dioctadecyl-3,3,3'3'-tetra-methylindocyanide percholorate (Dil)-labeled oxLDL uptake was measured in PE-A channel.

**Table 2 Active ingredients used in this study.**

| ***Compound*** | ***Concentration*** |
|---|---|
| Vitamin D3 | 100nM |
| Lycopene | 0.5uM |
| Monacolin K | 10uM |
| Lycopene + Monacolin K | 0.5uM/ 10uM |
| Vitamin D3 + Monacolin K | 100nM/ 10uM |
| Lycopene + Vitamin D3 | 0.5uM/ 10uM |
| Monacolin K + Lycopene + Vitamin D3 | 10uM/ 0.5uM/ 100nM |

### Example 2: Inhibition of oxLDL uptake by macrophages under the influence of 3 active ingredients.

Macrophages were exposed to vitamin D3, lycopene and/or monacolin K and the inhibitory effect of oxLDL uptake of these three active ingredients was measured. The results of 6 independent measurements are shown in table 3. The inhibitory potentiol of each compound and composition was expressed as the difference between the measurements obtained with and without the compound or composition. The inhibitory potential of the compounds and compositions is shown in figure 1.

**Table 3; Inhibitory effect of three compounds on the uptake of oxLDL by macrophages.**

| **Active ingredient** | **oxLDL uptake [Median Fluorescence intensity]** | **Average** |
|---|---|---|
| Control | 12600 | |
| Control | 13300 | |
| Control | 12500 | |
| Control | 11900 | |
| Control | 13800 | |
| Control | 11600 | 12617 |
| VitD3 (100nM) 1 | 13100 | |
| VitD3 (100nM) 2 | 13200 | |
| VitD3 (100nM) 3 | 13100 | |
| VitD3 (100nM) 4 | 12600 | |
| VitD3 (100nM) 5 | 10900 | |
| VitD3 (100nM) 6 | 10900 | 12300 |
| Lycopene (0'5uM) 1 | 9832 | |
| Lycopene (0'5uM) 2 | 11500 | |
| Lycopene (0'5uM) 3 | 12200 | |
| Lycopene (0'5uM) 4 | 11900 | |
| Lycopene (0'5uM) 5 | 11300 | |
| Lycopene (0'5uM) 6 | 11700 | 11405 |
| Monacolin K (10uM) 1 | 14300 | |
| Monacolin K (10uM) 2 | 11900 | |
| Monacolin K (10uM) 3 | 11900 | |
| Monacolin K (10uM) 4 | 11700 | |
| Monacolin K (10uM) 5 | 11800 | |
| Monacolin K (10uM) 6 | 14400 | 12667 |
| Monacolin K (10uM) + Lycopene (0'5uM) 1 | 11800 | |
| Monacolin K (10uM) + Lycopene (0'5uM) 2 | 12000 | |
| Monacolin K (10uM) + Lycopene (0'5uM) 3 | 10800 | |
| Monacolin K (10uM) + Lycopene (0'5uM) 4 | 13800 | |
| Monacolin K (10uM) + Lycopene (0'5uM) 5 | 12600 | |
| Monacolin K (10uM) + Lycopene (0'5uM) 6 | 11400 | 12067 |
| Monacolin K (10uM) + VitD3 (100nM) 1 | 12100 | |
| Monacolin K (10uM) + VitD3 (100nM) 2 | 12400 | |
| Monacolin K (10uM) + VitD3 (100nM) 3 | 11600 | |
| Monacolin K (10uM) + VitD3 (100nM) 4 | 12800 | |
| Monacolin K (10uM) + VitD3 (100nM) 5 | 10500 | |
| Monacolin K (10uM) + VitD3 (100nM) 6 | 10200 | 11600 |
| Lycopene (0'5uM) + VitD3 (100nM) 1 | 14500 | |
| Lycopene (0'5uM) + VitD3 (100nM) 2 | 11700 | |
| Lycopene (0'5uM) + VitD3 (100nM) 3 | 12200 | |
| Lycopene (0'5uM) + VitD3 (100nM) 4 | 11900 | |
| Lycopene (0'5uM) + VitD3 (100nM) 5 | 11000 | |
| Lycopene (0'5uM) + VitD3 (100nM) 6 | 9640 | 11823 |
| Monacolin K (10uM) + Lycopene (0'5uM) + VitD3 (100nM) 1 | 9876 | |
| Monacolin K (10uM) + Lycopene (0'5uM) + VitD3 (100nM) 2 | 9261 | |
| Monacolin K (10uM) + Lycopene (0'5uM) + VitD3 (100nM) 3 | 8222 | |
| Monacolin K (10uM) + Lycopene (0'5uM) + VitD3 (100nM) 4 | 9421 | |
| Monacolin K (10uM) + Lycopene (0'5uM) + VitD3 (100nM) 5 | 10300 | |
| Monacolin K (10uM) + Lycopene (0'5uM) + VitD3 (100nM) 6 | 10500 | 9597 |

### Reference List

1. Wald NJ, MR Law, A strategy to reduce cardiovascular disease by more than 80%. BMJ 326, 1419 (Jun 28, 2003).
2. Bogsrud MP et al., HypoCol (red yeast rice) lowers plasma cholesterol - a randomized placebo controlled study. Scandinavian cardiovascular journal : SCJ 44, 197-200 (Aug, 2010).
3. Yang CW, SA Mousa, The effect of red yeast rice (Monascus purpureus) in dyslipidemia and other disorders. Complementary therapies in medicine 20, 466-74 (Dec, 2012).
4. Liu J et al., Chinese red yeast rice (Monascus purpureus) for primary hyperlipidemia: a meta-analysis of randomized controlled trials. Chinese medicine 1, 4 (2006).
5. Heber D et al., Cholesterol-lowering effects of a proprietary Chinese red-yeast-rice dietary supplement. The American journal of clinical nutrition 69, 231-6 (Feb, 1999).
6. Gheith O et al., Efficacy and safety of Monascus purpureus Went rice in subjects with secondary hyperlipidemia. Clinical and experimental nephrology 12, 189-94 (Jun, 2008).
7. Lin CC et al., Efficacy and safety of Monascus purpureus Went rice in subjects with hyperlipidemia. European journal of endocrinology / European Federation of Endocrine Societies 153, 679-86 (Nov, 2005).
8. Li JJ et al., Impact of long-term Xuezhikang therapy on cardiovascular events in high-risk patients with nonspecific, preexisting abnormal liver tests: a post-hoc analysis from Chinese Coronary Secondary Prevention Study (CCSPS). International journal of cardiology 154, 362-5 (Feb 9, 2012).
9. Li JJ et al., Long-term effects of Xuezhikang on blood pressure in hypertensive patients with previous myocardial infarction: data from the Chinese Coronary Secondary Prevention Study (CCSPS). Clinical and experimental hypertension 32, 491-8 (2010).
10. Li JJ et al., Impact of Xuezhikang on coronary events in hypertensive patients with previous myocardial infarction from the China Coronary Secondary Prevention Study (CCSPS). Annals of medicine 42, 231-40 (Apr, 2010).
11. Li JJ et al., Beneficial impact of Xuezhikang on cardiovascular events and mortality in elderly hypertensive patients with previous myocardial infarction from the China Coronary Secondary Prevention Study (CCSPS). Journal of clinical pharmacology 49, 947-56 (Aug, 2009).
12. Lu ZL et al., [China coronary secondary prevention study (CCSPS): outcomes from analysis of coronary heart disease patients with diabetes]. Zhonghua xin xue guan bing za zhi 33, 1067-70 (Dec, 2005).
13. Lu ZL, [China coronary secondary prevention study (CCSPS)]. Zhonghua xin xue guan bing za zhi 33, 109-15 (Feb, 2005).
14. Yin J et al., Efficacy of berberine in patients with type 2 diabetes mellitus. Metabolism: clinical and experimental 57, 712-7 (May, 2008).
15. Zhang Y et al., Treatment of type 2 diabetes and dyslipidemia with the natural plant alkaloid berberine. The Journal of clinical endocrinology and metabolism 93, 2559-65 (Jul, 2008).
16. Zhang H et al., Berberine lowers blood glucose in type 2 diabetes mellitus patients through increasing insulin receptor expression. Metabolism: clinical and experimental 59, 285-92 (Feb, 2010).
17. Kong W et al., Berberine is a novel cholesterol-lowering drug working through a unique mechanism distinct from statins. Nature medicine 10, 1344-51 (Dec, 2004).
18. Kong WJ et al., Combination of simvastatin with berberine improves the lipid-lowering efficacy. Metabolism: clinical and experimental 57, 1029-37 (Aug, 2008).
19. Marazzi G et al., Long-term effects of nutraceuticals (berberine, red yeast rice, policosanol) in elderly hypercholesterolemic patients. Advances in therapy 28, 1105-13 (Dec, 2011).
20. Affuso F et al., Effects of a nutraceutical combination (berberine, red yeast rice and policosanols) on lipid levels and endothelial function randomized, double-blind, placebo-controlled study. Nutrition, metabolism, and cardiovascular diseases: NMCD 20, 656-61 (Nov, 2010).
21. Affuso F et al., A nutraceutical combination improves insulin sensitivity in patients with metabolic syndrome. World journal of cardiology 4, 77-83 (Mar 26, 2012).
22. Trimarco B et al., Clinical evidence of efficacy of red yeast rice and berberine in a large controlled study versus diet. Mediterranean journal of nutrition and metabolism 4, 133-139 (Aug, 2011).
23. Wang JM et al., Berberine-induced decline in circulating CD31 +/CD42-microparticles is associated with improvement of endothelial function in humans. European journal of pharmacology 614, 77-83 (Jul 1, 2009).
24. Gu Y et al., Effect of traditional Chinese medicine berberine on type 2 diabetes based on comprehensive metabonomics. Talanta 81, 766-72 (May 15, 2010).
25. Karppi J et al., Serum beta-carotene and the risk of sudden cardiac death in men: A population-based follow-up study. Atherosclerosis, (Nov 8, 2012).
26. Karppi J et al., Serum lycopene decreases the risk of stroke in men: a population-based follow-up study. Neurology 79, 1540-7 (Oct 9, 2012).
27. Palozza P et al., Effect of Lycopene and Tomato Products on Cholesterol Metabolism. Annals of nutrition & metabolism 61, 126-134 (Sep 8, 2012).
28. Ried K, P Fakler, Protective effect of lycopene on serum cholesterol and blood pressure: Meta-analyses of intervention trials. Maturitas 68, 299-310 (Apr, 2011).
29. McEneny J et al., Lycopene intervention reduces inflammation and improves HDL functionality in moderately overweight middle-aged individuals. The Journal of nutritional biochemistry 24, 163-8 (Jan, 2013).
30. Ho MJ et al., Blood pressure lowering efficacy of coenzyme Q10 for primary hypertension. Cochrane database of systematic reviews, CD007435 (2009).
31. Rosenfeldt FL et al., Coenzyme Q10 in the treatment of hypertension: a meta-analysis of the clinical trials. Journal of human hypertension 21, 297-306 (Apr, 2007).
32. Tran MT et al., Role of coenzyme Q10 in chronic heart failure, angina, and hypertension. Pharmacotherapy 21, 797-806 (Jul, 2001).
33. Gao L et al., Effects of coenzyme Q10 on vascular endothelial function in humans: a meta-analysis of randomized controlled trials. Atherosclerosis 221, 311-6 (Apr, 2012).
34. Thibault A et al., Phase I study of lovastatin, an inhibitor of the mevalonate pathway, in patients with cancer. Clinical cancer research : an official journal of the American Association for Cancer Research 2, 483-91 (Mar, 1996).
35. Kim WS et al., Phase II study of high-dose lovastatin in patients with advanced gastric adenocarcinoma. Investigational new drugs 19, 81-3 (2001).
36. Caso G et al., Effect of coenzyme q10 on myopathic symptoms in patients treated with statins. The American journal of cardiology 99, 1409-12 (May 15, 2007).
37. Zlatohlavek L et al., The effect of coenzyme Q10 in statin myopathy. Neuro endocrinology letters 33, 98-101 (Nov 28, 2012).
38. Jia Y et al., Could ginseng-based medicines be better than nitrates in treating ischemic heart disease? A systematic review and meta-analysis of randomized controlled trials. Complementary therapies in medicine 20, 155-66 (Jun, 2012).
39. Han KH et al., Effect of red ginseng on blood pressure in patients with essential hypertension and white coat hypertension. The American journal of Chinese medicine 26, 199-209 (1998).
40. Sung J et al., Effects of red ginseng upon vascular endothelial function in patients with essential hypertension. The American journal of Chinese medicine 28, 205-16 (2000).
41. Rhee MY et al., Effect of Korean red ginseng on arterial stiffness in subjects with hypertension. Journal of alternative and complementary medicine 17, 45-9 (Jan, 2011).
42. Kim JY et al., Beneficial effects of Korean red ginseng on lymphocyte DNA damage, antioxidant enzyme activity, and LDL oxidation in healthy participants: a randomized, double-blind, placebo-controlled trial. Nutrition journal 11, 47 (2012).
43. Kantor ED et al., Association between use of specialty dietary supplements and C-reactive protein concentrations. American journal of epidemiology 176, 1002-13 (Dec 1, 2012).
44. Yi SW et al., Association between ginseng intake and mortality: Kangwha cohort study. Journal of alternative and complementary medicine 15, 921-8 (Aug, 2009).
45. McGreevy C, D Williams, New insights about vitamin D and cardiovascular disease: a narrative review. Annals of internal medicine 155, 820-6 (Dec 20, 2011).
46. Forman JP et al., Plasma 25-hydroxyvitamin D levels and risk of incident hypertension among young women. Hypertension 52, 828-32 (Nov, 2008).
47. Forman JP et al., Plasma 25-hydroxyvitamin D levels and risk of incident hypertension. Hypertension 49, 1063-9 (May, 2007).
48. Burgaz A et al., Confirmed hypertension and plasma 25(OH)D concentrations amongst elderly men. Journal of internal medicine 269, 211-8 (Feb, 2011).
49. Jorde R et al., Serum 25-hydroxyvitamin D levels are strongly related to systolic blood pressure but do not predict future hypertension. Hypertension 55, 792-8 (Mar, 2010).
50. Giovannucci E et al., 25-hydroxyvitamin D and risk of myocardial infarction in men: a prospective study. Archives of internal medicine 168, 1174-80 (Jun 9, 2008).
51. Wang L et al., Circulating 25-hydroxy-vitamin d and risk of cardiovascular disease: a meta-analysis of prospective studies. Circulation. Cardiovascular quality and outcomes 5, 819-29 (Nov 1, 2012).
52. Karakas M et al., Low Levels of Serum 25-Hydroxyvitamin D Are Associated with Increased Risk of Myocardial Infarction, Especially in Women: Results from the MONICA/KORA Augsburg Case-Cohort Study. The Journal of clinical endocrinology and metabolism, (Nov 12, 2012).
53. Lavie CJ et al., Vitamin D status, left ventricular geometric abnormalities and cardiovascular disease. Journal of internal medicine, (Nov 2, 2012).
54. Wang TJ et al., Vitamin D deficiency and risk of cardiovascular disease. Circulation 117, 503-11 (Jan 29, 2008).
55. Grandi NC et al., Vitamin D and cardiovascular disease: systematic review and meta-analysis of prospective studies. Preventive medicine 51, 228-33 (Sep-Oct, 2010).
56. Bostick RM et al., Relation of calcium, vitamin D, and dairy food intake to ischemic heart disease mortality among postmenopausal women. American journal of epidemiology 149, 151-61 (Jan 15, 1999).
57. Pfeifer M et al., Effects of a short-term vitamin D(3) and calcium supplementation on blood pressure and parathyroid hormone levels in elderly women. The Journal of clinical endocrinology and metabolism 86, 1633-7 (Apr, 2001).
58. Tamez H et al., Vitamin D reduces left atrial volume in patients with left ventricular hypertrophy and chronic kidney disease. American heart journal 164, 902-909 e2 (Dec, 2012).
59. Fallo F et al., Low serum 25-hydroxyvitamin D levels are associated with left ventricular hypertrophy in essential hypertension. Nutrition, metabolism, and cardiovascular diseases : NMCD 22, 871-6 (Oct, 2012).
60. Dong Y et al., A 16-week randomized clinical trial of 2000 international units daily vitamin D3 supplementation in black youth: 25-hydroxyvitamin D, adiposity, and arterial stiffness. The Journal of clinical endocrinology and metabolism 95, 4584-91 (Oct, 2010).
61. Harris RA et al., Vitamin D3 supplementation for 16 weeks improves flow-mediated dilation in overweight African-American adults. American journal of hypertension 24, 557-62 (May, 2011).
62. Carrelli AL et al., Vitamin D deficiency is associated with subclinical carotid atherosclerosis: the Northern Manhattan study. Stroke; a journal of cerebral circulation 42, 2240-5 (Aug, 2011).
63. Geleijnse JM et al., Dietary intake of menaquinone is associated with a reduced risk of coronary heart disease: the Rotterdam Study. The Journal of nutrition 134, 3100-5 (Nov, 2004).
64. Gast GC et al., A high menaquinone intake reduces the incidence of coronary heart disease. Nutrition, metabolism, and cardiovascular diseases : NMCD 19, 504-10 (Sep, 2009).
65. Sato Y et al., The prevention of hip fracture with menatetrenone and risedronate plus calcium supplementation in elderly patients with Alzheimer disease: a randomized controlled trial. The Kurume medical journal 57, 117-24 (2011).
66. Binkley N et al., Vitamin K treatment reduces undercarboxylated osteocalcin but does not alter bone turnover, density, or geometry in healthy postmenopausal North American women. Journal of bone and mineral research : the official journal of the American Society for Bone and Mineral Research 24, 983-91 (Jun, 2009).
67. Vermeer C et al., Beyond deficiency: potential benefits of increased intakes of vitamin K for bone and vascular health. European journal of nutrition 43, 325-35 (Dec, 2004).
68. Cranenburg EC et al., Vitamin K: the coagulation vitamin that became omnipotent. Thrombosis and haemostasis 98, 120-5 (Jul, 2007).
69. Beulens JW et al., High dietary menaquinone intake is associated with reduced coronary calcification. Atherosclerosis 203, 489-93 (Apr, 2009).
70. Zhang L et al., Effects of statin therapy on inflammatory markers in chronic heart failure: a meta-analysis of randomized controlled trials. Archives of medical research 41, 464-71 (Aug, 2010).
71. Kinlay S, Low-density lipoprotein-dependent and -independent effects of cholesterol-lowering therapies on C-reactive protein: a meta-analysis. Journal of the American College of Cardiology 49, 2003-9 (May 22, 2007).
72. Kwak B et al., Statins as a newly recognized type of immunomodulator. Nature medicine 6, 1399-402 (Dec, 2000).
73. Janda S et al., The effect of statins on mortality from severe infections and sepsis: a systematic review and meta-analysis. Journal of critical care 25, 656 e7-22 (Dec, 2010).
74. Tleyjeh IM et al., Statins for the prevention and treatment of infections: a systematic review and meta-analysis. Archives of internal medicine 169, 1658-67 (Oct 12, 2009).
75. Mihaylova B et al., The effects of lowering LDL cholesterol with statin therapy in people at low risk of vascular disease: meta-analysis of individual data from 27 randomised trials. Lancet 380, 581-90 (Aug 11, 2012).
76. Feng Y et al., Natural polypill Xuezhikang: its clinical benefit and potential multicomponent synergistic mechanisms of action in cardiovascular disease and other chronic conditions. Journal of alternative and complementary medicine 18, 318-28 (Apr, 2012).
77. Lin YL et al., Biologically active components and nutraceuticals in the Monascus-fermented rice: a review. Applied microbiology and biotechnology 77, 965-73 (Jan, 2008).
78. Cicero AF et al., Antihyperlipidaemic effect of a Monascus purpureus brand dietary supplement on a large sample of subjects at low risk for cardiovascular disease: a pilot study. Complementary therapies in medicine 13, 273-8 (Dec, 2005).
79. Keithley JK et al., A pilot study of the safety and efficacy of cholestin in treating HIV-related dyslipidemia. Nutrition 18, 201-4 (Feb, 2002).
80. Golomb BA, MA Evans, Statin adverse effects : a review of the literature and evidence for a mitochondrial mechanism. American journal of cardiovascular drugs: drugs, devices, and other interventions 8, 373-418 (2008).
81. Nijjar PS et al., Role of dietary supplements in lowering low-density lipoprotein cholesterol: a review. Journal of clinical lipidology 4, 248-58 (Jul-Aug, 2010).
82. Lapi F et al., Myopathies associated with red yeast rice and liquorice: spontaneous reports from the Italian Surveillance System of Natural Health Products. British journal of clinical pharmacology 66, 572-4 (Oct, 2008).
83. Mueller PS, Symptomatic myopathy due to red yeast rice. Annals of internal medicine 145, 474-5 (Sep 19, 2006).
84. Smith DJ, KE Olive, Chinese red rice-induced myopathy. Southern medical journal 96, 1265-7 (Dec, 2003).
85. Grieco A et al., Acute hepatitis caused by a natural lipid-lowering product: when "alternative" medicine is no "alternative" at all. Journal of hepatology 50, 1273-7 (Jun, 2009).
86. Wigger-Alberti W et al., Anaphylaxis due to Monascus purpureus-fermented rice (red yeast rice). Allergy 54, 1330-1 (Dec, 1999).
87. Eisenbrand G, Toxicological evaluation of red mould rice. Molecular nutrition & food research 50, 322-7 (Mar, 2006).
88. Li CL, YF.; Hou, ZL., Xuezhikang toxicity study. Bull Chinese Pharmacol Soc 12, (1995).
89. Li C et al., Monascus purpureus-fermented rice (red yeast rice): A natural food product that lowers blood cholesterol in animal models of hypercholesterolemia. Nutrition Research 18, 71-81 (1998).
90. Halbert SC et al., Tolerability of red yeast rice (2,400 mg twice daily) versus pravastatin (20 mg twice daily) in patients with previous statin intolerance. The American journal of cardiology 105, 198-204 (Jan 15, 2010).
91. Venero CV et al., Lipid-lowering efficacy of red yeast rice in a population intolerant to statins. The American journal of cardiology 105, 664-6 (Mar 1, 2010).
92. Prasad GV et al., Rhabdomyolysis due to red yeast rice (Monascus purpureus) in a renal transplant recipient. Transplantation 74, 1200-1 (Oct 27, 2002).
93. Vercelli L et al., Chinese red rice depletes muscle coenzyme Q10 and maintains muscle damage after discontinuation of statin treatment. Journal of the American Geriatrics Society 54, 718-20 (Apr, 2006).
94. Roselle H et al., Symptomatic hepatitis associated with the use of herbal red yeast rice. Annals of internal medicine 149, 516-7 (Oct 7, 2008).
95. Dong H et al., Berberine in the treatment of type 2 diabetes mellitus: a systemic review and meta-analysis. Evidence-based complementary and alternative medicine : eCAM 2012, 591654 (2012).
96. Marin-Neto JA et al., Cardiovascular effects of berberine in patients with severe congestive heart failure. Clinical cardiology 11, 253-60 (Apr, 1988).
97. Zeng XH et al., Efficacy and safety of berberine for congestive heart failure secondary to ischemic or idiopathic dilated cardiomyopathy. The American journal of cardiology 92, 173-6 (Jul 15, 2003).
98. Affuso F et al., Cardiovascular and metabolic effects of Berberine. World journal of cardiology 2, 71-7 (Apr 26, 2010).
99. Li Y et al., Bioactivities of berberine metabolites after transformation through CYP450 isoenzymes. Journal of translational medicine 9, 62 (2011).
100. Cicero AF et al., Eulipidemic effects of berberine administered alone or in combination with other natural cholesterol-lowering agents. A single-blind clinical investigation. Arzneimittel-Forschung 57, 26-30 (2007).
101. Cao Y, Shandong University of Traditional Chinese Medicine (2008).
102. Li Z, LH Liu, "Therapeutic efficacy of combined berberine and glipizide on type 2 diabetes". Journal of Clinical Research 24, 61-64 (2007).
103. Wang W, Shanxi Medical University (2008).
104. Wenping Y, Clinical efficacy of berberine treatment of diabetes. Modern Hospital 10, 9-10 (2010).
105. Sabir M, NK Bhide, Study of some pharmacological actions of berberine. Indian journal of physiology and pharmacology 15, 111-32 (Jul, 1971).
106. Yuan J et al., [Effect of berberine on transit time of human small intestine]. Zhongguo Zhong xi yi jie he za zhi Zhongguo Zhongxiyi jiehe zazhi = Chinese journal of integrated traditional and Western medicine / Zhongguo Zhong xi yi jie he xue hui, Zhongguo Zhong yi yan jiu yuan zhu ban 14, 718-20 (Dec, 1994).
107. Kupeli E et al., A comparative study on the anti-inflammatory, antinociceptive and antipyretic effects of isoquinoline alkaloids from the roots of Turkish Berberis species. Life sciences 72, 645-57 (Dec 27, 2002).
108. Chan E, Displacement of bilirubin from albumin by berberine. Biology of the neonate 63, 201-8 (1993).
109. Enerback L, Berberine sulphate binding to mast cell polyanions: a cytofluorometric method for the quantitation of heparin. Histochemistry 42, 301-13 (1974).
110. Xin HW et al., The effects of berberine on the pharmacokinetics of cyclosporin A in healthy volunteers. Methods and findings in experimental and clinical pharmacology 28, 25-9 (Jan-Feb, 2006).
111. Lin HL et al., Berberine modulates expression of mdr1 gene product and the responses of digestive track cancer cells to Paclitaxel. British journal of cancer 81, 416-22 (Oct, 1999).
112. Vrzal R et al., Activation of the aryl hydrocarbon receptor by berberine in HepG2 and H4IIE cells: Biphasic effect on CYP1A1. Biochemical pharmacology 70, 925-36 (Sep 15, 2005).
113. Kardinaal AF et al., Association between beta-carotene and acute myocardial infarction depends on polyunsaturated fatty acid status. The EURAMIC Study. European Study on Antioxidants, Myocardial Infarction, and Cancer of the Breast. Arteriosclerosis, thrombosis, and vascular biology 15, 726-32 (Jun, 1995).
114. Sesso HD et al., Plasma lycopene, other carotenoids, and retinol and the risk of cardiovascular disease in women. The American journal of clinical nutrition 79, 47-53 (Jan, 2004).
115. Sesso HD et al., Dietary lycopene, tomato-based food products and cardiovascular disease in women. The Journal of nutrition 133, 2336-41 (Jul, 2003).
116. Palozza P et al., Lycopene induces cell growth inhibition by altering mevalonate pathway and Ras signaling in cancer cell lines. Carcinogenesis 31, 1813-21 (Oct, 2010).
117. Palozza P et al., Lycopene regulation of cholesterol synthesis and efflux in human macrophages. The Journal of nutritional biochemistry 22, 971-8 (Oct, 2011).
118. Burton-Freeman B et al., Protective activity of processed tomato products on postprandial oxidation and inflammation: a clinical trial in healthy weight men and women. Molecular nutrition & food research 56, 622-31 (Apr, 2012).
119. Giovannucci E et al., Intake of carotenoids and retinol in relation to risk of prostate cancer. Journal of the National Cancer Institute 87, 1767-76 (Dec 6, 1995).
120. Etminan M et al., The role of tomato products and lycopene in the prevention of prostate cancer: a meta-analysis of observational studies. Cancer epidemiology, biomarkers & prevention : a publication of the American Association for Cancer Research, cosponsored by the American Society of Preventive Oncology 13, 340-5 (Mar, 2004).
121. Ilic D et al., Lycopene for the prevention of prostate cancer. Cochrane database of systematic reviews, CD008007 (2011).
122. Giovannucci E, Commentary: Serum lycopene and prostate cancer progression: a re-consideration of findings from the prostate cancer prevention trial. Cancer causes & control : CCC 22, 1055-9 (Jul, 2011).
123. Kristal AR et al., Serum lycopene concentration and prostate cancer risk: results from the Prostate Cancer Prevention Trial. Cancer epidemiology, biomarkers & prevention : a publication of the American Association for Cancer Research, cosponsored by the American Society of Preventive Oncology 20, 638-46 (Apr, 2011).
124. Hu F et al., Carotenoids and breast cancer risk: a meta-analysis and meta-regression. Breast cancer research and treatment 131, 239-53 (Jan, 2012).
125. Mohanty NK et al., Lycopene as a chemopreventive agent in the treatment of high-grade prostate intraepithelial neoplasia. Urologic oncology 23, 383-5 (Nov-Dec, 2005).
126. Forbes K et al., Lycopene increases urokinase receptor and fails to inhibit growth or connexin expression in a metastatically passaged prostate cancer cell line: a brief communication. Experimental biology and medicine 228, 967-71 (Sep, 2003).
127. Sesso HD et al., Dietary and plasma lycopene and the risk of breast cancer. Cancer epidemiology, biomarkers & prevention : a publication of the American Association for Cancer Research, cosponsored by the American Society of Preventive Oncology 14, 1074-81 (May, 2005).
128. Kavanaugh CJ et al., The U.S. Food and Drug Administration's evidence-based review for qualified health claims: tomatoes, lycopene, and cancer. Journal of the National Cancer Institute 99, 1074-85 (Jul 18, 2007).
129. Aune D et al., Dietary compared with blood concentrations of carotenoids and breast cancer risk: a systematic review and meta-analysis of prospective studies. The American journal of clinical nutrition 96, 356-73 (Aug, 2012).
130. Kabat GC et al., Longitudinal study of serum carotenoid, retinol, and tocopherol concentrations in relation to breast cancer risk among postmenopausal women. The American journal of clinical nutrition 90, 162-9 (Jul, 2009).
131. Dorjgochoo T et al., Plasma carotenoids, tocopherols, retinol and breast cancer risk: results from the Shanghai Women Health Study (SWHS). Breast cancer research and treatment 117, 381-9 (Sep, 2009).
132. Mortensen SA et al., Dose-related decrease of serum coenzyme Q10 during treatment with HMG-CoA reductase inhibitors. Molecular aspects of medicine 18 Suppl, S137-44 (1997).
133. Ghirlanda G et al., Evidence of plasma CoQ10-lowering effect by HMG-CoA reductase inhibitors: a double-blind, placebo-controlled study. Journal of clinical pharmacology 33, 226-9 (Mar, 1993).
134. Colquhoun DM et al., Effects of simvastatin on blood lipids, vitamin E, coenzyme Q10 levels and left ventricular function in humans. European journal of clinical investigation 35, 251-8 (Apr, 2005).
135. Watts GF et al., Plasma coenzyme Q (ubiquinone) concentrations in patients treated with simvastatin. Journal ofclinical pathology 46, 1055-7 (Nov, 1993).
136. Lamperti C et al., Muscle coenzyme Q10 level in statin-related myopathy. Archives of neurology 62, 1709-12 (Nov, 2005).
137. Paiva H et al., High-dose statins and skeletal muscle metabolism in humans: a randomized, controlled trial. Clinical pharmacology and therapeutics 78, 60-8 (Jul, 2005).
138. Young JM et al., Effect of coenzyme Q(10) supplementation on simvastatin-induced myalgia. The American journal of cardiology 100, 1400-3 (Nov 1, 2007).
139. Bookstaver DA et al., Effect of coenzyme Q10 supplementation on statin-induced myalgias. The American journal of cardiology 110, 526-9 (Aug 15, 2012).
140. Fotino AD et al., Effect of coenzyme Q10 supplementation on heart failure: a meta-analysis. The American journal of clinical nutrition 97, 268-75 (Feb, 2013).
141. Greenberg S, WH Frishman, Co-enzyme Q10: a new drug for cardiovascular disease. Journal of clinical pharmacology 30, 596-608 (Jul, 1990).
142. Hidaka T et al., Safety assessment of coenzyme Q10 (CoQ10). BioFactors 32, 199-208 (2008).
143. Mousa SA, Antithrombotic effects of naturally derived products on coagulation and platelet function. Methods in molecular biology 663, 229-40 (2010).
144. Hodgson JM et al., Coenzyme Q10 improves blood pressure and glycaemic control: a controlled trial in subjects with type 2 diabetes. European journal of clinical nutrition 56, 1137-42 (Nov, 2002).
145. Pepping J, Coenzyme Q10. American journal of health-system pharmacy : AJHP : official journal of the American Society of Health-System Pharmacists 56, 519-21 (Mar 15, 1999).
146. Mancini A et al., Relationships between plasma CoQ10 levels and thyroid hormones in chronic obstructive pulmonary disease. BioFactors 25, 201-4 (2005).
147. Wee JJ et al., in Herbal Medicine: Biomolecular and Clinical Aspects, IFF Benzie, S Wachtel-Galor, Eds. (Boca Raton (FL), 2011).
148. Yun TK, Experimental and epidemiological evidence on non-organ specific cancer preventive effect of Korean ginseng and identification of active compounds. Mutation research 523-524, 63-74 (Feb-Mar, 2003).
149. Yun TK, Panax ginseng--a non-organ-specific cancer preventive? The lancet oncology 2, 49-55 (Jan, 2001).
150. Geng J et al., Ginseng for cognition. Cochrane database of systematic reviews, CD007769 (2010).
151. Lee MS et al., Ginseng for cognitive function in Alzheimer's disease: a systematic review. Journal of Alzheimer's disease : JAD 18, 339-44 (2009).
152. Jang DJ et al., Red ginseng for treating erectile dysfunction: a systematic review. British journal of clinical pharmacology 66, 444-50 (Oct, 2008).
153. An X et al., Oral ginseng formulae for stable chronic obstructive pulmonary disease: a systematic review. Respiratory medicine 105, 165-76 (Feb, 2011).
154. Jovanovski E et al., Effects of Korean red ginseng (Panax ginseng C.A. Mayer) and its isolated ginsenosides and polysaccharides on arterial stiffness in healthy individuals. American journal of hypertension 23, 469-72 (May, 2010).
155. Kim S et al., Red ginseng for type 2 diabetes mellitus: a systematic review of randomized controlled trials. Chinese journal of integrative medicine 17, 937-44 (Dec, 2011).
156. Vuksan V et al., American ginseng (Panax quinquefolius L.) attenuates postprandial glycemia in a time-dependent but not dose-dependent manner in healthy individuals. The American journal of clinical nutrition 73, 753-8 (Apr, 2001).
157. Vuksan V et al., American ginseng (Panax quinquefolius L) reduces postprandial glycemia in nondiabetic subjects and subjects with type 2 diabetes mellitus. Archives of internal medicine 160, 1009-13 (Apr 10, 2000).
158. Kim SY et al., Effects of red ginseng supplementation on menopausal symptoms and cardiovascular risk factors in postmenopausal women: a double-blind randomized controlled trial. Menopause 19, 461-6 (Apr, 2012).
159. Lee NH, CG Son, Systematic review of randomized controlled trials evaluating the efficacy and safety of ginseng. Journal of acupuncture and meridian studies 4, 85-97 (Jun, 2011).
160. Engels HJ et al., Effects of ginseng supplementation on supramaximal exercise performance and short-term recovery. Journal of strength and conditioning research / National Strength & Conditioning Association 15, 290-5 (Aug, 2001).
161. Allen JD et al., Ginseng supplementation does not enhance healthy young adults' peak aerobic exercise performance. Journal of the American College of Nutrition 17, 462-6 (Oct, 1998).
162. Engels HJ, JC Wirth, No ergogenic effects of ginseng (Panax ginseng C.A. Meyer) during graded maximal aerobic exercise. Journal of the American Dietetic Association 97, 1110-5 (Oct, 1997).
163. Morris AC et al., No ergogenic effect of ginseng ingestion. International journal of sport nutrition 6, 263-71 (Sep, 1996).
164. Caron MF et al., Electrocardiographic and hemodynamic effects of Panax ginseng. The Annals of pharmacotherapy 36, 758-63 (May, 2002).
165. Vuksan V et al., Korean red ginseng (Panax ginseng) improves glucose and insulin regulation in well-controlled, type 2 diabetes: results of a randomized, double-blind, placebo-controlled study of efficacy and safety. Nutrition, metabolism, and cardiovascular diseases : NMCD 18, 46-56 (Jan, 2008).
166. Markham ØMAaKR, Ed., Flavonoids: Chemistry, Biochemistry and Applications, (CRC Press 2005), pp. 471 -551.
167. Corder R et al., Oenology: red wine procyanidins and vascular health. Nature 444, 566 (Nov 30, 2006).
168. Havsteen B, Flavonoids, a class of natural products of high pharmacological potency. Biochemical pharmacology 32, 1141-8 (Apr 1, 1983).
169. Hertog MG et al., Intake of potentially anticarcinogenic flavonoids and their determinants in adults in The Netherlands. Nutrition and cancer 20, 21-9 (1993).
170. Lotito SB, B Frei, Consumption of flavonoid-rich foods and increased plasma antioxidant capacity in humans: cause, consequence, or epiphenomenon? Free radical biology & medicine 41, 1727-46 (Dec 15, 2006).
171. Wallace TC, Anthocyanins in cardiovascular disease. Advances in nutrition 2, 1-7 (Jan, 2011).
172. Godycki-Cwirko M et al., Uric acid but not apple polyphenols is responsible for the rise of plasma antioxidant activity after apple juice consumption in healthy subjects. Journal of the American College of Nutrition 29, 397-406 (Aug, 2010).
173. Sumner MD et al., Effects of pomegranate juice consumption on myocardial perfusion in patients with coronary heart disease. The American journal of cardiology 96, 810-4 (Sep 15, 2005).
174. Aviram M et al., Pomegranate juice consumption for 3 years by patients with carotid artery stenosis reduces common carotid intima-media thickness, blood pressure and LDL oxidation. Clinical nutrition 23, 423-33 (Jun, 2004).
175. Gorinstein S et al., Red grapefruit positively influences serum triglyceride level in patients suffering from coronary atherosclerosis: studies in vitro and in humans. Journal of agricultural and food chemistry 54, 1887-92 (Mar 8, 2006).
176. Naruszewicz M et al., Combination therapy of statin with flavonoids rich extract from chokeberry fruits enhanced reduction in cardiovascular risk markers in patients after myocardial infraction (MI). Atherosclerosis 194, e179-84 (Oct, 2007).
177. Karlsen A et al., Anthocyanins inhibit nuclear factor-kappaB activation in monocytes and reduce plasma concentrations of pro-inflammatory mediators in healthy adults. The Journal ofnutrition 137, 1951-4 (Aug, 2007).
178. Kelley DS et al., Consumption of Bing sweet cherries lowers circulating concentrations of inflammation markers in healthy men and women. The Journal of nutrition 136, 981-6 (Apr, 2006).
179. Scarabelli TM et al., Targeting STAT1 by myricetin and delphinidin provides efficient protection of the heart from ischemia/reperfusion-induced injury. FEBS letters 583, 531-41 (Feb 4, 2009).
180. Peterson JJ et al., Associations between flavonoids and cardiovascular disease incidence or mortality in European and US populations. Nutrition reviews 70, 491-508 (Sep, 2012).
181. Mink PJ et al., Flavonoid intake and cardiovascular disease mortality: a prospective study in postmenopausal women. The American journal of clinical nutrition 85, 895-909 (Mar, 2007).
182. Kay CD et al., Relative impact of flavonoid composition, dose and structure on vascular function: A systematic review of randomised controlled trials of flavonoid-rich food products. Molecular nutrition & food research 56, 1605-16 (Nov, 2012).
183. McCullough ML et al., Flavonoid intake and cardiovascular disease mortality in a prospective cohort of US adults. The American journal of clinical nutrition 95, 454-64 (Feb, 2012).
184. Di Castelnuovo A et al., Meta-analysis of wine and beer consumption in relation to vascular risk. Circulation 105, 2836-44 (Jun 18, 2002).
185. van Velden DP et al., Red wines good, white wines bad? Redox report: communications in free radical research 7, 315-6 (2002).
186. Renaud S, M de Lorgeril, Wine, alcohol, platelets, and the French paradox for coronary heart disease. Lancet 339, 1523-6 (Jun 20, 1992).
187. van Dam RM et al., Potentially modifiable determinants of vitamin D status in an older population in the Netherlands: the Hoorn Study. The American journal of clinical nutrition 85, 755-61 (Mar, 2007).
188. Passeri G et al., Calcium metabolism and vitamin D in the extreme longevity. Experimental gerontology 43, 79-87 (Feb, 2008).
189. Hypponen E, C Power, Hypovitaminosis D in British adults at age 45 y: nationwide cohort study of dietary and lifestyle predictors. The American journal of clinical nutrition 85, 860-8 (Mar, 2007).
190. Wang L et al., Systematic review: Vitamin D and calcium supplementation in prevention of cardiovascular events. Annals of internal medicine 152, 315-23 (Mar 2, 2010).
191. Sokol SI et al., The effects of vitamin D repletion on endothelial function and inflammation in patients with coronary artery disease. Vascular medicine 17, 394-404 (Dec, 2012).
192. Elamin MB et al., Vitamin D and cardiovascular outcomes: a systematic review and meta-analysis. The Journal of clinical endocrinology and metabolism 96, 1931-42 (Jul, 2011).
193. Pittas AG et al., Systematic review: Vitamin D and cardiometabolic outcomes. Annals of internal medicine 152, 307-14 (Mar 2, 2010).
194. Witham MD et al., Effect of vitamin D on blood pressure: a systematic review and meta-analysis. Journal of hypertension 27, 1948-54 (Oct, 2009).
195. Gage BF et al., Risk of osteoporotic fracture in elderly patients taking warfarin: results from the National Registry of Atrial Fibrillation 2. Archives of internal medicine 166, 241-6 (Jan 23, 2006).
196. Schurgers LJ et al., Oral anticoagulant treatment: friend or foe in cardiovascular disease? Blood 104, 3231-2 (Nov 15, 2004).
197. Spronk HM et al., Tissue-specific utilization of menaquinone-4 results in the prevention of arterial calcification in warfarin-treated rats. Journal of vascular research 40, 531-7 (Nov-Dec, 2003).
198. Choi HJ et al., Vitamin K2 supplementation improves insulin sensitivity via osteocalcin metabolism: a placebo-controlled trial. Diabetes care 34, e147 (Sep, 2011).

## Claims

1. Composition for oral administration having a beneficial effect on the cardiovascular system, comprising as active ingredients a mixture of monacolin K, lycopene and vitamin D3.

2. Composition according to claim 1 further comprising an active ingredient selected from the group consisting of Berberine, coenzyme Q10, panax ginseng, flavonoids, vitamin K2, folic acid, vitamin B, biotin and a mineral.

3. Composition according to claim 2 wherein the mineral is selected from the group consisting of Calcium, Magnesium, Selenium, Zinc, Iodine or Chromium.

4. Composition according to any one of claims 1 - 3 in the form of a unit dose.

5. Composition according to any one of claims 1 - 4 comprising between 2 and 40 mg of monacolin K per unit dose, preferably about 10 mg per unit dose.

6. Composition according to any one of claims 1 - 5 comprising between and 100 mg of lycopene per unit dose, preferably about 25 mg per unit dose.

7. Composition according to any one of claims 1 - 6 comprising between 1 and 100 microgram of vitamin D3 per unit dose, preferably about 25 microgram per unit dose.

8. Composition according to any one of claims 1 - 7 wherein the unit dose comprises a pill, sachet, capsule, bulk powder container, tablet or equivalent suited for oral administration to a subject.

9. Composition according to any one of claims 1 - 8 for use as a medicament.

10. Composition according to any one of claims 1 - 8 for use in the treatment of a disease selected from the group consisting of heart disease, cardiovascular disease, hypercholesterolaemia, hypertriglyceridaemia, hyperlipidemia, type II diabetes, elevated blood pressure, hypertension, pre-diabetes, myocardial infarction, stroke, heart failure, chronic heart failure and congestive heart failure.

## Patentansprüche

1. Zusammensetzung zur oralen Verabreichung mit günstiger Wirkung auf das Kardiovaskulare System, umfassend, als Wirkstoffe, eine Mischung von Monacolin K, Lycopen und Vitamin D3.

2. Zusammensetzung nach Anspruch 1, weiterhin umfassend einen Wirkstoff ausgewählt aus der Gruppe bestehend aus Berberin, Coenzym Q10, Panax-Ginseng, Flavonoiden, Vitamin K2, Folsäure, Vitamin B, Biotin und einem Mineral.

3. Zusammensetzung nach Anspruch 2, wobei das Mineral ausgewählt ist aus der Gruppe bestehend aus Calcium, Magnesium, Selen, Zink, Iod und Chrom.

4. Zusammensetzung nach einem der Ansprüche 1-3 in Form einer Einheitsdosis.

5. Zusammensetzung nach einem der Ansprüche 1-4, umfassend zwischen 2 und 40 mg Monacolin K pro Einheitsdosis, vorzugsweise etwa 10 mg pro Einheitsdosis.

6. Zusammensetzung nach einem der Ansprüche 1-5, umfassend zwischen 1 und 100 mg Lycopen pro Einheitsdosis, vorzugsweise etwa 25 mg pro Einheitsdosis.

7. Zusammensetzung nach einem der Ansprüche 1-6, umfassend zwischen 1 und 100 µg Vitamin D3 pro Einheitsdosis, vorzugsweise etwa 25 µg pro Einheitsdosis.

8. Zusammensetzung nach einem der Ansprüche 1-7, wobei die Einheitsdosis eine Pille, einen Beutel, eine Kapsel, einen Behälter für eine Pulvermasse, eine Tablette oder ein für die orale Verabreichung an ein Subjekt geeignetes Äquivalent umfasst.

9. Zusammensetzung nach einem der Ansprüche 1-8 zur Verwendung als Medikament.

10. Zusammensetzung nach einem der Ansprüche 1-8 zur Verwendung bei der Behandlung einer Krankheit ausgewählt aus der Gruppe bestehend aus Herzkrankheit, Herz-Kreislauf-Krankheit, Hypercholesterinämie, Hypertriglyzeridämie, Hyperlipidämie, Typ-II-Diabetes, erhöhtem Blutdruck, Hypertonie, Prädiabetes, Herzinfarkt, Schlaganfall, Herzinsuffizienz, chronischer Herzinsuffizienz und dekompensierter Herzinsuffizienz.

## Revendications

1. Composition pour administration orale ayant un effet avantageux sur le système cardiovasculaire, comprenant au titre de principes actifs un mélange de monacoline K, de lycopène et de vitamine D3.

2. Composition selon la revendication 1, comprenant en outre un principe actif choisi dans le groupe constitué par les suivants : Berbérine, coenzyme Q10, panax ginseng, flavonoïdes, vitamine K2, acide folique, vitamine B, biotine et un minéral.

3. Composition selon la revendication 2, où le minéral est choisi dans le groupe constitué par les suivants : calcium, magnésium, sélénium, zinc, iode ou chrome.

4. Composition selon l'une quelconque des revendications 1 à 3 sous la forme d'une dose unitaire.

5. Composition selon l'une quelconque des revendications 1 à 4 comprenant entre 2 et 40 mg de monacoline K par dose unitaire, préférentiellement environ 10 mg par dose unitaire.

6. Composition selon l'une quelconque des revendications 1 à 5 comprenant entre 1 et 100 mg de lycopène par dose unitaire, préférentiellement environ 25 mg par dose unitaire.

7. Composition selon l'une quelconque des revendications 1 à 6 comprenant entre 1 et 100 mg de vitamine D3 par dose unitaire, préférentiellement environ 25 microgrammes par dose unitaire.

8. Composition selon l'une quelconque des revendications 1 à 7, où la dose unitaire est constituée d'une pilule, d'un sachet d'une capsule, d'un récipient de poudre en vrac, d'un comprimé ou équivalent, adaptés à l'administration orale à un sujet.

9. Composé selon l'une quelconque des revendications 1 à 8, pour utilisation en tant que médicament.

10. Composition selon l'une quelconque des revendications 1 à 8, pour utilisation dans le traitement d'une maladie choisie dans le groupe constitué par les suivantes : cardiopathie, maladie cardiovasculaire, hypercholestérolémie, hypertriglycéridémie, hyperlipidémie, diabète de type II, tension artérielle élevée, hypertension, prédiabète, infarctus du myocarde, accident vasculaire cérébral, insuffisance cardiaque, insuffisance cardiaque chronique et insuffisance cardiaque congestive.
